# FASCICULE DE BREVET EUROPEEN

(11) **EP 1 425 263 B1**
(45) Date de publication et mention de la délivrance du brevet: **15.12.2010**
(21) Numéro de dépôt: 02774907.6
(22) Date de dépôt: 06.09.2002
(51) Int. Cl.: C07C 235/12, C22B 60/02

(54) **COMPOSES THERMOSENSIBLES PRESENTANT UN POINT DE TROUBLE, UTILISABLES COMME EXTRACTANTS POUR LA SEPARATION DE METAUX EN SOLUTION AQUEUSE**
THERMISCH EMPFINDLICHE ZUSAMMENSETZUNGEN MIT EINEM TRÜBUNGSPUNKT ZUR VERWENDUNG ALS EXTRAKTIONSMITTEL ZUR ABTRENNUNG VON METALLEN AUS WÄSSRIGEN MEDIEN
HEAT-SENSITIVE COMPOUNDS HAVING A CLOUD POINT WHICH CAN BE USED AS EXTRACTANTS FOR THE SEPARATION OF METALS IN AQUEOUS SOLUTION

(30) Priorité: 10.09.2001 FR 0111665
(43) Date de publication de la demande: 09.06.2004
(73) Titulaire: Commissariat à l'Énergie Atomique et aux Énergies Alternatives, 75015 Paris (FR)
(72) Inventeur: MADIC, Charles, F-94320 Thiais (FR); BERTHON, Laurence, F-30330 Connaux (FR); ZORZ, Nicole, F-30200 Bagnols S/Ceze (FR); COULOMBEAU, Hélène, 35700 Rennes (FR); TESTARD, Fabienne, F-75013 Paris (FR); ZEMB, Thomas, F-78220 Viroflay (FR); GIBANEL, Sébastien, F-24130 La Force (FR); LARPENT, Chantal, F-78000 Versailles (FR); BACZKO, Krystyna, F-91400 Orsay (FR)
(74) Mandataire: Poulin, Gérard
(86) Numéro de dépôt international: PCT/FR2002/003038
(87) Numéro de publication internationale: WO 2003/022798

(56) Documents cités:
- WO-A-00/73521
- US-A- 4 154 674
- K. TAKESHITA ET AL.: "Extraction of lanthanide elements by thermosensitive polymer gel copolymerized with acidic phosphorus compound" SOLVENT EXTRACTION AND ION EXCHANGE, vol. 18, no. 2, 2000, pages 375-386, XP008003473 cité dans la demande
- D.E. BERGBREITER ET AL.: "Sequestration of Trace Metals Using Water-Soluble and Fluorous Phase-Soluble Polymers" ANGEWANDTE CHEMIE. INTERNATIONAL EDITION., vol. 39, no. 6, 2000, pages 1039-1042, XP002199521 VERLAG CHEMIE. WEINHEIM., DE ISSN: 0570-0833 cité dans la demande

## Description

### Domaine technique

L'invention concerne un composé thermosensible présentant un point de trouble, c'est-à-dire ayant la propriété d'être soluble dans l'eau en dessous d'une température critique T_{c} (appelée également point de trouble) et insoluble dans l'eau au-dessus de cette température T_{c}, cette propriété étant thermoréversible.

De tels composés peuvent être utilisés en particulier comme extractants pour la séparation d'espèces chimiques, en particulier de métaux tels que les actinides et les lanthanides, ou de tout autre élément non métallique, par action d'un stimulus externe qui est une variation de température.

L'espèce chimique peut être en particulier sous forme d'ions simples (1 seul atome) ou d'ions complexes constitués par un groupe d'atomes.

### Etat de la technique antérieure

On a déjà envisagé d'utiliser des composés thermosensibles pour la séparation de métaux comme il est décrit par Bergbreiter D.E., Koshti N., Franchina J.G., Frels J.G., Angew. Chem. Int. Ed., 39, 2000, 1040-2 [1] et par Takeshita K. et Nakano Y., Solvent Extraction and Ion Exchange, 18(2), 2000, 375-86 [2].

Dans la référence [1], on utilise comme composé thermosensible un copolymère de N-isopropyl acrylamide et de N-acryloxy succinimide (PNIPAM-c-NASI) lié à de l'acide hydroxamique, qui présente une température critique de 31°C. Ce composé thermosensible peut être utilisé pour séparer des ions Fe^{III}. Dans ce cas, on l'ajoute à la solution aqueuse contenant les ions Fe^{III}, ce qui conduit à une solution homogène de couleur rouge caractéristique du complexe ferrique. Lorsqu'on augmente la température au-dessus de 31°C, on obtient une précipitation du polymère coloré laissant un surnageant incolore. Le dosage des ions ferriques dans le surnageant montre que plus de 99% des ions ferriques ont été séquestrés dans le polymère précipité, soit une capacité de séquestration du polymère de l'ordre de 2 mg de Fe^{III} par gramme de polymère dans l'exemple donné.

Dans la référence [2], on utilise un composé thermosensible constitué par un gel de copolymère de N-isopropyl acrylamide (NIPA) et de 2-méthacryloyloxyéthylphosphate acide pour extraire des lanthanides d'une solution aqueuse contenant des ions nitrate. En solution aqueuse, ces copolymères forment des gels qui se déshydratent et se rétractent progressivement par augmentation de température piégeant ainsi l'ion à séparer. Typiquement, les expériences ont été réalisées sur 50 ml de solution aqueuse contenant 500 ppm de La^{(III)} ou de Sm^{(III)} et 50 mg de composé thermosensible, soit un rapport massique ion/polymère de 1/2.

Dans la référence [1], on peut aussi utiliser un copolymère PNIPAM-c-NASI lié à la 3-hydroxy-2-pyridinone pour séparer Fe^{III} et Cu^{(II)} en utilisant le même principe.

On connaît aussi des procédés d'extraction au moyen de solutions micellaires de tensioactifs non ioniques, présentant un point de trouble, utilisés principalement en chimie analytique pour l'extraction de composés organiques, de protéines ou autres biomolécules, et de cations métalliques, comme il est décrit par Hinze W.L., Pramauro E., Critical Reviews in Analytical Chemistry, 24(2), 1993, 133-77 [3] et par Tani H., Kamidate T., Watanabe H., J. Chromatogr. A, 780, 1997, 229-241 [4].

Le principe de ces extractions est basé sur :
(1) la solubilisation du soluté à extraire dans les micelles de tensioactifs en solution aqueuse, à une température inférieure au point de trouble T_{c}, et
(2) la séparation de phase, démixtion, induite par augmentation de la température au-dessus de T_{c} : le soluté solubilisé dans les micelles est entraîné avec le tensioactif dans la phase concentrée.

Un des intérêts majeurs de cette technique est la concentration. L'efficacité de l'extraction dépend de l'affinité du soluté pour les micelles de tensioactifs (coefficient de partage micelles/phase aqueuse) : elle est beaucoup plus élevée pour des solutés hydrophobes que pour des solutés polaires hydrophiles. L'extraction d'ions métalliques, ayant peu d'affinité avec les tensioactifs courants, peut être réalisée en introduisant dans le système un agent chélatant organique qui a pour rôle de complexer l'ion métallique et de permettre sa solubilisation dans les micelles sous forme de chélate métallique, plus hydrophobe, puis sa séparation ultérieure par augmentation de température. L'efficacité de la séparation dépend de l'hydrophobie de l'agent chélatant et du complexe.

Dans la plupart des cas, les travaux ont été conduits avec un objectif d'analyse de traces, l'extraction par point de trouble étant utilisée comme étape de pré-concentration.

Les travaux décrits traitent de la séparation de cations de métaux de transition Ni^{II}, Zn^{II}, Cu^{II}, Co^{II} et de cation d'uranyle U^{VI} en faibles concentrations.

Les tensioactifs utilisés sont le plus souvent des alkylphériolpolyéthoxylés (Triton X114, X100, PONPE dont les T_{c} sont respectivement de l'ordre de 25, 65 et 5°C) à des concentrations de l'ordre de 0,1 à 0,5% en masse, bien supérieures à leur concentration micellaire critique. Les rapports de concentration molaire en tensioactif par rapport au cation sont très élevés : de l'ordre de 50 à 1 000.

Les agents chélatants utilisés sont des complexants bien connus de ces ions comme par exemple le 1-(2-pyridylazo)-2-naphtol (PAN) pour la séparation de Zn^{II} ou U^{VI}, des dérivés de 2-(2-thiazoloylazo)-4-phenol différemment substitués sur le phénol tels que TAC (CH₃), TAMP (OCH₃), TAEP (OC₂H₅) et TAPP (C₆H₅) pour la séparation de Ni^{II}, Zn^{II}, Fe^{II} ou Cd^{II}.

Par ailleurs, il a été proposé dans US-A-4,154,674 [12] d'utiliser, pour extraire sélectivement des cations métalliques d'une solution, des dérivés de polyoxyalkylène glycols, et notamment des dérivés dans lesquels la chaîne polyoxyalkylène est liée à un polymère du type polystyrène ou un copolymère du styrène et d'un autre monomère comme le divinylbenzène.

Il a également été proposé dans WO-A-00/73521 [13] de séparer les lanthanides des actinides présents dans un milieu aqueux par nanofiltration-complexation, en utilisant des ligands de type polyacides amines.

### Exposé de l'invention

La présente invention a précisément pour objet de nouveaux composés thermosensibles, qui peuvent être utilisés comme extractants de diverses espèces chimiques, par exemple de métaux tels que les actinides et les lanthanides, sous forme d'ions métalliques simples ou complexes, et de toute autre espèce chimique non métallique, en mettant en oeuvre une étape de complexation de l'espèce chimique par le composé thermosensible, à une température inférieure à la température critique T_{c} de ce composé, puis une seconde étape de séparation du complexe formé, espèce chimique/composé thermosensible, par augmentation de la température à une température supérieure à T_{c}.

Selon l'invention, le composé thermosensible a la propriété d'être soluble dans l'eau en dessous d'une température critique T_{c} et insoluble dans l'eau au-dessus de cette température T_{c}, cette propriété étant thermoréversible. Le composé comprend une première partie amphiphile et thermoréversible qui est dérivée d'un tensioactif non-ionique polyéthoxylé et qui répond à l'une des formules (I) et (II) suivantes : 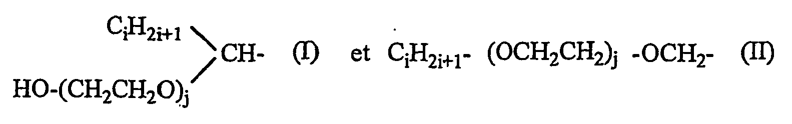 dans lesquelles i est un nombre entier allant de 1 à 20 et j est un nombre entier allant de 3 à 30, cette première partie étant liée chimiquement à une seconde partie capable de complexer une espèce chimique.

Ainsi, la première partie du composé thermosensible selon l'invention est dérivée d'un tensioactif non-ionique polyéthoxylé de formule R-O(CH₂CH₂O)ₙ-H dans laquelle R représente une chaîne alkyle linéaire ou ramifiée, et n est un nombre entier allant de 3 à 30.

Des tensioactifs de ce type sont décrits par exemple dans Huibers P.D.T., Shah D.O., Katrizky A.R., J. Colloid and Interface Sci., 193, 1997, 132-6 [5]. Ils présentent un point de trouble qui peut se situer dans une large gamme allant de 6 à 100°C.

Ce point de trouble, qui va déterminer la température critique du composé thermosensible, dépend de la structure du tensioactif. Ainsi, le point de trouble augmente avec le nombre de motifs éthoxy et diminue avec la longueur de la chaîne alkyle.

Ces tensioactifs non-ioniques sont classiquement notés CᵢEⱼ, i représentant le nombre d'atomes de carbone de la chaîne alkyle et j représentant le nombre de groupes éthoxy.

Le choix du tensioactif non-ionique permet donc de contrôler la température critique du composé thermosensible.

La seconde partie du composé thermosensible qui présente la propriété de complexer une espèce chimique, par exemple un métal ou un ion métallique simple ou complexe, peut avoir une structure monomérique, oligomérique ou polymérique. Elle comprend avantageusement un acide aminé, un peptide, un oligopeptide, ou un polypeptide.

A titre d'exemple d'acide aminé utilisable, on peut citer la lysine qui permet de complexer l'uranium sous forme d'ions uranyle.

Lorsque la seconde partie comprend un oligomère ou un polymère autre qu'un oligopeptide ou un polypeptide, il peut s'agir d'un polymère comportant un ou plusieurs groupements complexants du type amine, amide ou acide, par exemple d'une polyamine, d'un polyamide ou d'un polyacide.

A titre d'exemple, la seconde partie peut répondre à la formule (III) suivante : 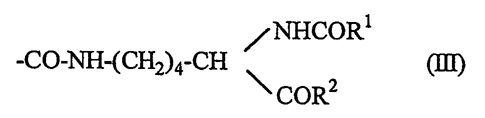 dans laquelle R¹ représente un groupe hydrocarboné et R² représente un groupe hydroxyle, un groupe alcoxy ou un groupement azoté substitué ou non.

Le groupe hydrocarboné utilisé pour R¹ a de préférence de 1 à 10 atomes de carbone ; ce groupe hydrocarboné peut être un groupe alkyle.

Lorsque R² représente un groupe azoté, ce groupe peut répondre à l'une des formules suivantes : NH₂, NHR et NR₂ avec R représentant un groupe hydrocarboné, de préférence alkyle, ayant de 1 à 10 atomes de carbone.

Lorsque R² représente un groupe alcoxy, celui-ci a de préférence 1 à 10 atomes de carbone.

A titre d'exemple, R¹ peut représenter le groupe méthyle et R² peut représenter le groupe hydroxyle ou le groupe méthoxy.

A titre d'exemples de composés thermosensibles conformes à l'invention, on peut citer :
- le composé répondant à la formule (IV) suivante : 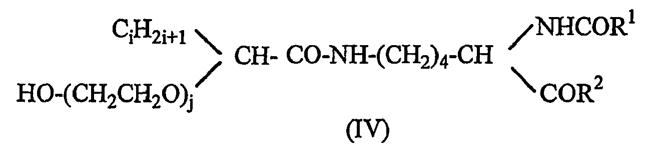 dans laquelle i est égal à 12, j est égal à 4, R¹ représente CH₃ et R² représente OCH₃, et
- les composés répondant à la formule (V) suivante : 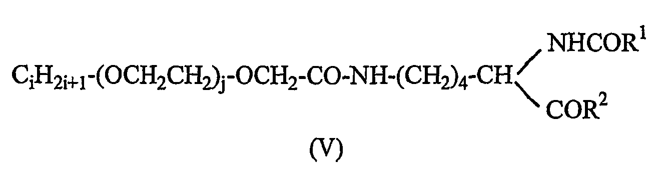 dans laquelle i est égal à 12, j est égal à 5, R¹ représente CH₃ et R² représente OCH₃ ou OH.

Les composés thermosensibles de l'invention possèdent la propriété d'être solubles dans l'eau en dessous de la température critique T_{c} et de devenir insolubles dans l'eau et solubles dans les huiles (solvants organiques, hydrocarbures) au-dessus de cette température critique.

La valeur de la température critique dépend de la concentration en composé thermosensible et de la force ionique du milieu.

En ce qui concerne la concentration, elle a peu d'influence sur la température critique des composés thermosensibles de l'invention. En effet, les variations sont inférieures à 5°C sur une gamme de concentration massique allant de 0,5 à 20%.

En ce qui concerne la force ionique du milieu, celle-ci exerce une influence légèrement plus forte sur la température critique T_{c}.

Ainsi, dans le cas où le milieu comprend des nitrates alcalins, la température critique augmente avec la polarité du cation alcalin. Ceci se traduit par une augmentation en présence de nitrate de lithium et une faible variation en présence de nitrate de sodium.

De préférence, les composés thermosensibles de l'invention présentent une température critique T_{c} de 20 à 90°C, à une concentration massique de 0,1 à 25% en solution aqueuse.

Selon l'invention, on peut contrôler et moduler la température critique T_{c} du composé en choisissant un tensioactif non-ionique présentant un point de trouble approprié.

Ainsi, dans le cas des composés de formule (IV) où la chaîne alkyle est greffée en α et laisse le groupement polyéthoxylé non substitué en bout de chaîne, on a une forte augmentation du point de trouble

(50-55°C) par rapport au point de trouble (6°C) du squelette CᵢEⱼ du tensioactif correspondant.

Au contraire, dans le cas des composés de formule (V) où la chaîne alkyle est située à l'extrémité du groupement polyéthoxylé, le point de trouble est très proche de celui du squelette CᵢEⱼ correspondant.

Lorsque le composé répond à la formule (IV) donnée ci-dessus, il présente une température critique T_{c} de 55 à 75°C, à une concentration massique de 0,5 à 25% en solution aqueuse.

Lorsque les composés thermosensibles répondent à la formule (V) donnée ci-dessus, ils présentent une température critique T_{c} de 30 à 70°C, à une concentration massique de 0,1 à 25% en solution aqueuse.

L'invention a également pour objet un procédé de préparation d'un composé thermosensible, ayant la propriété d'être soluble dans l'eau en dessous d'une température critique T_{c} et insoluble dans l'eau au-dessus de cette température T_{c}, cette propriété étant thermoréversible, qui comprend la réaction d'un dérivé carboxylique répondant à l'une des formules (VI) et (VIII) suivantes : 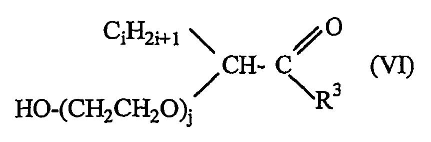 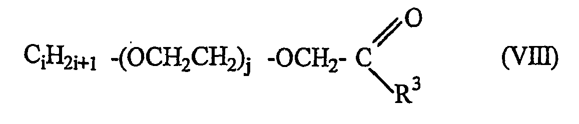 dans lesquelles i est un nombre entier allant de 1 à 20, j est un nombre entier allant de 3 à 30 et R³ représente Cl, OH ou un groupe alkoxy, avec une amine ou polyamine primaire ou secondaire comportant un groupe capable de complexer une espèce chimique.

L'amine ou la polyamine primaire ou secondaire peut être un acide aminé, un dérivé d'acide aminé, ou présenter une structure peptidique ou non peptidique.

Selon un premier mode de réalisation de l'invention, le procédé est utilisé pour la préparation d'un composé répondant à la formule (IV) suivante : 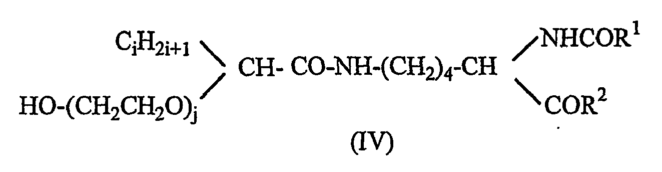 dans laquelle i est un nombre entier allant de 1 à 20, j est un nombre entier allant de 3 à 30, R¹ représente un groupe hydrocarboné et R² représente un groupe hydroxyle, un groupe alkoxy ou un groupement azoté, substitué ou non,
qui comprend la réaction d'un dérivé carboxylique répondant à la formule (VI) suivante : 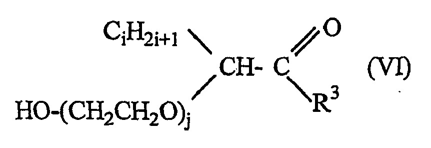 dans laquelle i et j sont tels que définis ci-dessus, et R³ représente Cl, OH ou un groupe alkoxy, avec le dérivé de lysine répondant à la formule (VII) suivante : 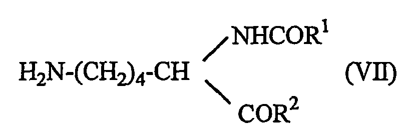 dans laquelle R¹ et R² sont tels que définis ci-dessus.

Dans ce mode de réalisation, le groupe hyroxyle du dérivé carboxylique de formule (VI) est de préférence protégé et le groupe carboxylique de ce dérivé est de préférence activé avant sa réaction avec le dérivé de lysine de formule (VII).

Le groupe hydroxyle peut être protégé sous forme d'éther, en particulier par une molécule du type tétrahydropyranne. L'activation du groupe carboxylique peut être réalisée au moyen de la N-hydroxysuccinimide.

Dans ce mode de réalisation, le procédé comprend en outre une étape de déprotection du groupe hydroxyle du dérivé de formule (VI) après réaction de celui-ci avec le dérivé de lysine de formule (VII).

Selon un second mode de réalisation du procédé de l'invention permettant la préparation d'un composé répondant à la formule (V) suivante : 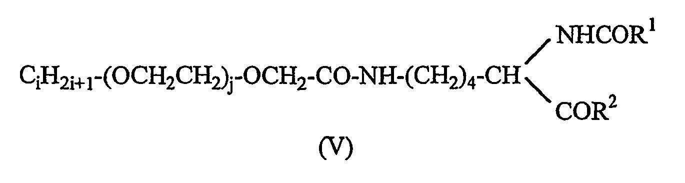 dans laquelle i est un nombre entier allant de 1 à 20, j est un nombre entier allant de 3 à 30, R¹ représente un groupe hydrocarboné et R² représente un groupe hydroxyle, un groupe alkoxy ou un groupement azoté, substitué ou non,
le procédé comprend la réaction d'un dérivé carboxylique répondant à la formule(VIII) suivante : 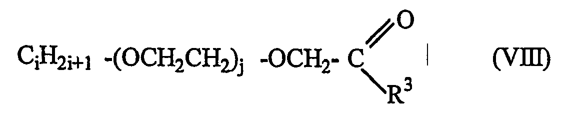 dans laquelle i et j sont tels que définis ci-dessus, et R³ représente Cl, OH ou un groupe alkoxy, avec le dérivé de lysine répondant à la formule (VII) suivante : 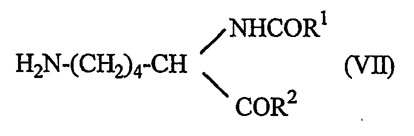 dans laquelle R¹ et R² sont tels que définis ci-dessus.

Dans le premier mode de réalisation du procédé, on peut obtenir le composé thermosensible en cinq étapes, à partir du polyoxyéthylène glycol de formule : HO-(CH₂CH₂O)ⱼ-H, avec des rendements globaux de l'ordre de 40%.

Dans la première étape, on protège une fonction alcool du polyoxyéthylène glycol, par exemple au moyen de dihydropyranne.

Dans la deuxième étape, on fait réagir le polyoxyéthylène glycol obtenu dans la première étape avec un acide bromo-alcanoïque de formule CᵢH₂ᵢ₊₁-CHBr-CO₂H pour obtenir le dérivé carboxylé de formule (VI) dans laquelle R³ peut représenter un groupe hydroxyle (OH).

Dans la troisième étape, on active ce dérivé carboxylique au moyen de N-hydroxysuccinimide.

Dans la quatrième étape, on fait réagir le dérivé carboxylique activé avec l'amine, le dérivé de lysine de formule (VII).

Dans la cinquième étape, on réalise une déprotection de la fonction hydroxyle du polyoxyéthylène glycol de départ.

Dans le second mode de réalisation du procédé de l'invention, le composé thermosensible peut être obtenu en deux étapes à partir du tensioactif non-ionique de formule : CᵢH₂ᵢ₊₁-(OCH₂CH₂)ⱼ-OH, par réaction de celui-ci avec l'acide bromoacétique, puis réaction du dérivé carboxylé avec l'amine, le dérivé de lysine de formule (VII).

Les composés thermosensibles de l'invention peuvent être utilisés pour séparer au moins une espèce chimique présente dans une solution aqueuse.

Aussi, l'invention a également pour objet un procédé d'extraction d'au moins une espèce chimique présente dans une solution aqueuse, en système monophasique aqueux, qui comprend les étapes suivantes :
a) ajouter à la solution aqueuse un composé thermosensible tel que défini ci-dessus, à une température inférieure à la température critique T_{c} de ce composé,
b) porter le milieu réactionnel à une température supérieure à la température critique T_{c} du composé pour séparer le milieu réactionnel en deux phases, constituées respectivement par une phase concentrée enrichie en l'espèce chimique à extraire, de densité supérieure à celle de l'eau, qui sédimente, et en une phase aqueuse diluée surnageante, appauvrie en l'espèce chimique, et
c) séparer la phase concentrée en l'espèce chimique du milieu réactionnel.

Selon l'invention, la séparation de cette phase peut être obtenue en système monophasique aqueux par simple décantation de la phase concentrée enrichie en l'espèce chimique à extraire.

Selon une variante de réalisation du procédé d'extraction, utilisant un système diphasique, ce procédé comprend les étapes suivantes :
a) ajouter à la solution aqueuse un composé thermosensible tel que défini ci-dessus, et une phase organique immiscible à l'eau, à une température inférieure à la température critique T_{c} de ce composé,
b) porter le milieu réactionnel à une température supérieure à la température critique T_{c} pour transférer le composé thermosensible et l'espèce chimique à extraire dans la phase organique, et
c) séparer la phase organique contenant l'espèce chimique du milieu réactionnel.

On peut ainsi réaliser la séparation en système biphasique liquide-liquide en ajoutant une phase liquide organique, par exemple un hydrocarbure non miscible, à la solution aqueuse à traiter contenant le composé thermosensible à une température inférieure à T_{c}. Par augmentation de la température du milieu réactionnel à une température supérieure à T_{c}, on provoque le transfert du composé thermosensible et de l'espèce chimique complexée par ce composé de la phase aqueuse vers la phase organique que l'on peut ensuite facilement séparer par simple décantation.

Les composés thermosensibles de l'invention permettent de complexer, dans une première étape, l'espèce chimique à extraire, puis de séparer le complexe espèce chimique-composé thermosensible, dans une seconde étape, par simple action d'un stimulus externe : l'augmentation de la température à une température supérieure à T_{c}.

Pour mettre en oeuvre le procédé d'extraction de l'invention, on utilise dans l'étape a) une quantité de composé thermosensible telle que la concentration massique de la solution aqueuse en composé thermosensible soit de 0,1 à 25%.

Ce procédé peut être utilisé pour séparer différentes espèces chimiques, notamment des métaux tels que les actinides et les lanthanides. Il peut être utilisé en particulier pour extraire l'uranium présent sous forme d'ion uranyle dans une solution aqueuse.

L'invention présente de nombreux avantages.

En effet, la structure en deux parties des composés thermosensibles utilisés permet d'affiner, d'une part, les températures de séparation par le choix du tensioactif thermoréversible et, d'autre part, les propriétés de complexation (aussi bien en terme de constante de complexation que de sélectivité vis-à-vis d'une espèce donnée) par le choix de la seconde partie complexante.

Par ailleurs, pour l'extraction des espèces chimiques, les composés hydrosolubles thermosensibles présentent les intérêts suivants :
1) la dissociation des deux étapes de complexation et de séparation :
   La solubilité des composés dans l'eau permet de les introduire directement dans la phase aqueuse à traiter. L'étape de complexation a lieu en milieu homogène : sa cinétique n'est donc pas limitée par des phénomènes de diffusion et de contact interfacial comme dans le cas des extractants lipophiles utilisés dans les processus actuels de séparation en milieu biphasique liquide-liquide.
   La durée de l'étape de complexation peut être facilement modulée.
2) La séparation est provoquée par action d'un stimulus externe : l'augmentation de température.
3) La limitation des volumes : le volume de phase enrichie en espèce chimique qui sédimente, ne représente que quelques % du volume de la phase aqueuse initiale.
   En terme de performances, les composés hydrosolubles thermosensibles développés ici sont efficaces pour la séparation de l'uranium sous forme de cation uranyle pour des rapports molaires extractant/uranium faibles : 0,5 à 1. Leurs capacités d'extraction sont supérieures ou du même ordre de grandeur que celles décrites avec les polymères complexants thermosensibles dérivés du PNIPAM.
   La structure en deux parties des composés qui associe les deux propriétés, complexation et thermoréversibilité, dans une même molécule permet d'obtenir des performances en extraction très supérieures.à celles obtenues à partir de mélanges d'un tensioactif thermoréversible et d'un complexant.
   De plus, on peut optimiser la structure des composés thermosensibles utilisés, notamment en ce qui concerne les propriétés de complexation.

Les performances en extraction peuvent être améliorées :
1) en introduisant dans la structure un complexant "fort" spécifique de l'espèce chimique à séparer,
2) en développant des structures oligomériques capables de mimer en une seule molécule les agrégats formés par les extractants classiques. Parmi les oligomères visés, on peut citer les peptides ou oligo-peptides comportant dans leur structure au moins une unité thermosensible de formule (I) ou (II) ainsi que les oligomères obtenus par greffage d'un tensioactif thermoréversible sur une structure macromoléculaire aminée ou polyaminée.

D'autres caractéristiques et avantages de l'invention apparaîtront mieux à la lecture de la description qui suit, d'exemples de réalisation donnés à titre illustratif et non limitatif, en référence à la figure unique annexée.

### Brève description du dessin

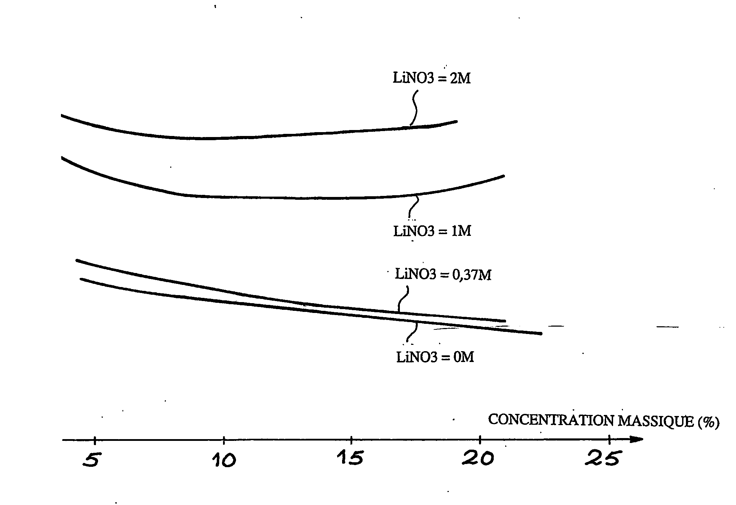

La figure 1 illustre, pour plusieurs concentrations en LiNO₃ d'une solution aqueuse, la variation de la température critique T_{c} (en °C) du composé la en fonction de sa concentration massique (en %).

### Exposé détaillé de modes de réalisation

### Exemple de synthèse du composé 1a :

Le composé **1a** est obtenu en 5 étapes à partir du diol polyéthoxylé **2** correspondant selon le schéma réactionnel suivant:

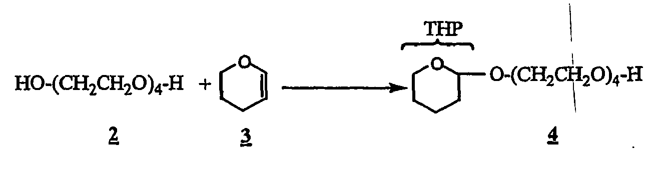

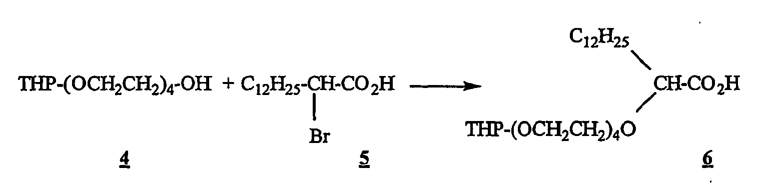

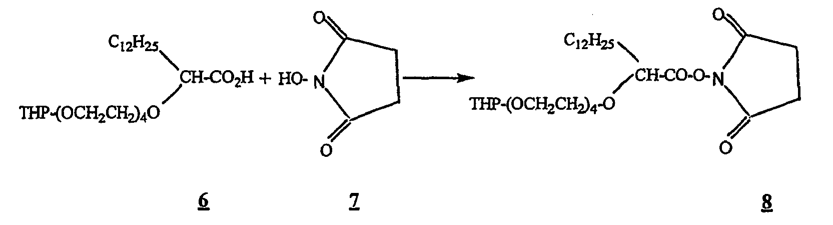

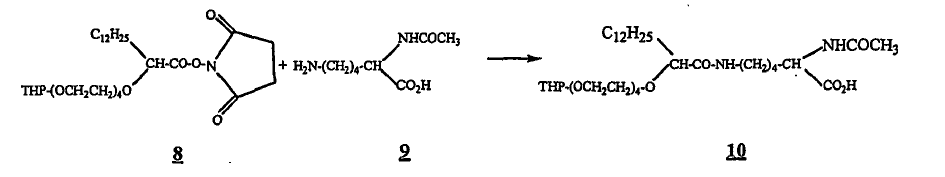

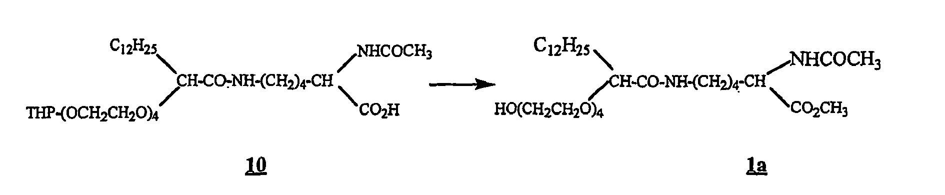

Dans la première étape, une fonction alcool du polyoxyéthylène glycol **2** est protégée selon une réaction décrite par Philippon et al. [6] pour donner l'alcool **4**. Cet alcool **4** est ensuite condensé avec l'acide-2 bromo-tétradécanoïque **5** pour donner l'acide **6** : la synthèse est adaptée du mode opératoire décrit par Wassermann et al. [7].

L'acide **6** est activé par réaction sur la N-hydroxysuccinimide **7** selon une synthèse adaptée du mode opératoire décrit par Popovitz-Biro et al. [8].

L'ester activé **8** est ensuite condensé sur la N-acétyl-lysine **9** pour donner **10** selon un mode opératoire adapté de la référence [8].

Le composé thermosensible **1a** est obtenu après déprotection de la fonction alcool de **10** et estérification selon un mode opératoire standard [9].

Le rendement global de la synthèse est de 37%.

### • 1ère étape : Préparation du composé 4 (E₄THP).

Le tétraéthylèneglycol **2** (30 g ; 163 mmoles), le dihydropyranne **3** (7,8 g ; 93 mmoles) et l'acide paratoluènesulfonique APTS (1,86 g ; 10,8 mmoles) sont dissous dans 54 ml de tétrahydrofuranne THF sec. Le mélange est agité à température ambiante. La disparition de **3** est suivie par chromatographie sur couche mince CCM, avec pour éluant le dichlorométhane CH₂Cl₂. Les plaques sont révélées par H₂SO₄. Dans ces conditions, **3** ne migre pas et **4** a un Rf de 0,39. La réaction est totale après 3h30. L'APTS est alors neutralisé par de la pyridine et le solvant est évaporé. Le résidu obtenu est repris au dichlorométhane et lavé 2 fois avec 20 ml d'eau puis une fois avec NaCl saturé. La phase organique est séchée sur Na₂SO₄ anhydre puis le solvant est évaporé.

Le produit **4** obtenu (25 g) est purifié en deux fois (2 x 12,5 g) par chromatographie sur une colonne de silice (350 g de silice, colonne de 6 cm de diamètre). L'éluant utilisé est composé successivement d'acétate d'éthyle 100% puis d'un mélange acétate d'éthyle 9/méthanol 1.
Masse obtenue = 15,70 g ; rendement = 61%
Composé **4** : [E₄THp]
Huile incolore
C₁₃H₂₆0₆
MM = 278 g/mol
RMN ¹H (CDCl₃) : 4,6 ppm (t, 1H, O-CH-O) ; 3,4-3,9 (m, 18 H, OCH₂CH₂ ; (CH₂)₃-CH₂-O) ; 1,5-1,8 (m, 6 H, CH-(CH₂)₃-CH₂O)

### • 2ème étape : Préparation du composé 6 [C₁₂CH(CO₂H)E₄THP]

Le composé **4** E₄THP (15,70 g ; 56 mmoles) et NaH (4,5 g d'une solution à 60% dans une huile minérale ; 187 mmoles) sont dilués dans 40 ml de THF anhydre (distillé sur CaH₂) et le mélange est agité à température ambiante pendant 30 minutes sous balayage d'azote. L'acide 2-bromotétradécanoique (17,40 g ; 56 mmoles) en solution dans 35 ml de THF est ajouté goutte à goutte au moyen d'une ampoule à brome. L'addition dure une heure. Le mélange réactionnel est alors chauffé avec un bain d'huile à 65°C. La réaction, suivie par CCM (éluant AcOEt 9/MeOH 1, révélation à l'iode), est totale après 24 heures. Le composé 6 ne migre quasiment pas et le Rf du composé **4** est de 0,57. Le mélange est repris avec 40 ml d'éther puis lavé 4 fois avec 250 ml de HCl 1N. La phase aqueuse est ensuite lavée à l'éther. Les phases organiques sont rassemblées puis lavées une fois avec 30 ml de NaCl jusqu'à pH 6. La phase organique obtenue est séchée sur Na₂SO₄ anhydre. Après évaporation du solvant, on récupère 28 g de produit **6** brut.

2,5 g de produit **6** brut sont purifiés par chromatographie sur colonne de silice (100 g) avec un gradient d'élution croissant : AcOEt/MeOH: 4/1 puis 1/1. On récupère 1,8 g de composé **6** pur. Rendement = 72%.
Composé **6** : [C₁₂CH(CO₂H)E₄THP]
Huile jaune
C₂₇H₅₂O₈
MM = 504 g/mol
RMN ¹H (CDCl₃) _{:} 4,6 ppm (t, 1H, O-CH-O) ; 3,4-4,1 (m, 19H, OCH₂CH₂, (CH₂)₃-CH₂OCH, (OCH₂CH₂)₅-CH) ; 1,1-2,4 (m, 28H, (CH₂)₁₁) ; 0,9 (t, 3H, CH₃(CH₂)₁₁).

### • 3ème étape : Préparation du composé 8 [C₁₂CH(CO₂NHS)E₄THP].

Le composé **6** (12,9 g ; 25,72 mmoles), la N-hydroxysuccinimide (3,22 g ; 28,29 mmoles) et le chlorhydrate de N-(3-diméthylaminopropyl)-N'-éthyl-carbodiimide EDC (5,5 g; 28,29 mmoles) sont dissous dans 30 ml de dichlorométhane sur tamis moléculaire. Le suivi de la réaction se fait par CCM avec l'éluant AcOEt 2/MeOH 1, avec une révélation par H₂SO₄. Dans ces conditions, le composé **6** a un Rf de 0, 50 et le composé **8** a un Rf de 0,66. Le mélange est agité une heure dans un bain de glace puis 20 heures à température ambiante. Le mélange est alors filtré sur célite afin d'éliminer le tamis moléculaire et le solvant est évaporé. Le mélange est repris au dichlorométhane et lavé une fois avec HCl 1 N puis 4 fois avec NaCl saturé jusqu'à pH 6. 15,4 g de produit sont ainsi obtenus et utilisés sans purification supplémentaire dans l'étape suivante.
Rendement quantitatif.
Composé **8** : [C₁₂CH(CO₂NHS)E₄THP)]
Huile jaune
C₃₁H₅₅O₁₀N
MM = 601 g/mol
RMN ¹H (CDCl₃) : 3,4-3,7 ppm (m, 19H, OCH₂CH₂, (OCH₂CH₂)₅-CH, (CH₂)₃-CH₂OCH) ; 1,2-1,9 (m, 28H, (CH₂)₁₁-, (CH₂)₃-CH₂OCH) ; 0,9 (t, 3H, CH₃(CH₂)₁₁-).

### • 4ème étape : Préparation du composé 10 [C₁₂CH(COAcLySCO₂H)E₄THP]

Le composé **8** brut (2,0 g ; 3,33 mmoles) est dissous dans 40 ml d'acétone. La N-acétyl-Lysine AcLysCO₂H (1,25 g ; 6,66 mmoles) et la triéthylamine (1,9 ml ; 13,32 mmoles) dissous dans 20 ml d'eau sont ajoutés au milieu réactionnel. Le suivi CCM se fait avec l'éluant AcOEt 4/MeOH 1/NH₃ 0,5 (révélation à la ninhydrine puis avec H₂SO₄). Le composé **8** a un Rf de 0,84 et le composé **10** a un Rf de 0,59. Après 20 heures d'agitation à température ambiante, la réaction est stoppée par addition d'acide trifluoroacétique et l'acétone est évaporée. Le mélange est repris avec 20 ml de dichlorométhane. L'acétyl-Lysine en excès est éliminée par extraction avec 15 ml d'une solution HCI 1N . La phase organique obtenue est lavée par 4 x 35 ml d'une solution saturée de NaCl jusqu'à pH 5. Après séchage sur Na₂SO₄ et évaporation du solvant, on récupère 2,0 g de composé **10** analytiquement pur.
Rendement = 89%
Huile jaune
C₃₅H₆₆O₁₀N₂
MM = 674 g/mol 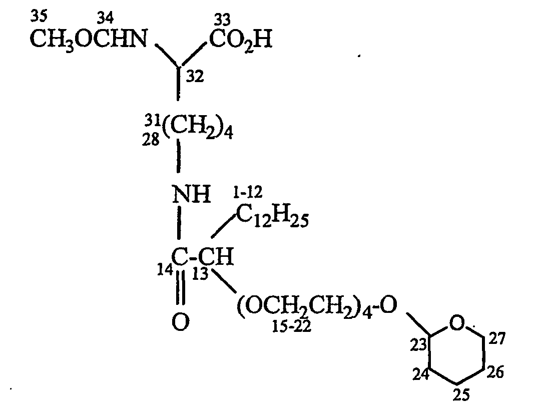 RMN ¹H (CDCl₃) _{:} 4,63 ppm (t, 1H, O-CH-O) ; 3,4-3,9(m, 22H, OCH₂(CH₂)₃, (CH₂)₁₁-CH-, (CH₂)NH-, (OCH₂CH₂)₄, (CH₂)₄-CH(CO₂H)N-) ; 2,0 (s, 3H, COCH₃) ; 1,1-1,6 (m, 34H, (CH₂)₁₁-, (CH₂)₃-CH₂OCH, NH-CH₂(CH₂)₃) ; 0,9 (t, 3H, (CH₂)₁₁-CH₃).
RMN ¹³C (CDCl₃) : 173,25 -173,5 ppm (C¹⁴-C³³) ; 98,65 (C²³) ; 66-70 (C¹⁵⁻²²) ; 61,9 (C¹⁵⁻²²) ; 52,9 (C³²) ; 51,9 - 32,74-31,47 (C²⁸) ; 28,7-30,4 - 21,48 - 19,06 (C²⁴⁻²⁶, C²⁸, C²⁻¹²) ; 13,67 (C¹).

### • 5ème étape : Synthèse du composé 1a [C₁₂CH(AcLysCO₂Me)E₄]

Le composé **10** (1,5 g ; 2,22 mmoles) et l'APTS (0,76 g ; 4,44 mmoles) sont dissous dans 10 ml de méthanol. Le mélange est agité à température ambiante. Le suivi CCM se fait avec l'éluant AcOEt 4/MeOH 1/NH₃ 0,5 (révélation à la ninhydrine puis avec H₂SO₄). Le composé **1a** a un Rf de 0,72. La réaction est totale après 5 heures. Le méthanol est alors évaporé. Le mélange est repris avec 15 ml de dichlorométhane et lavé avec 3 x 20 ml d'une solution saturée de NaCl jusqu'à pH neutre, afin d'éliminer l'APTS en excès. Après séchage sur Na₂SO₄ et évaporation du solvant, on récupère 1,2 g de produit.
Rendement = 89%
Huile jaune
C₃₁H₆₀O₉N₂
MM = 604 g/mol 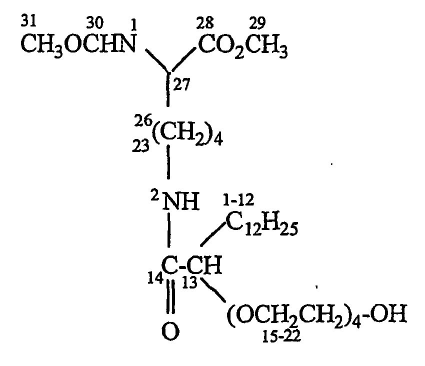 RMN ¹H (CDCl₃) : 7,3 ppm (d, NH (2)) ; 6,5 ppm (t, NH (1)) ; 4,51 ppm (1 H, C²⁷H) ;3,2-3,9 ppm (m, 22 H, (CH₂)₁₁-CH, CH₂-N-, (OCH₂CH₂)₄, CO₂CH₃) ; 2,0 ppm (s, 3H, NHCOCH₃) ; 1,2-1,8 ppm (m, 28 H, (CH₂)₁₁-, NH-CH₂(CH₂)₃) ; 0,9 ppm (t, 3H, (CH₂)₁₁-CH₃).
RMN ¹³C (CDCl₃) :172,82 ppm (CO) ; 172,79 ppm (CO) ; 172,65 ppm (CO) ; 172,58 ppm (CO) ; 144,58 ppm ; 122,96 ppm - 80,7 ppm - 69,3-72,0 ppm (C15-22) ; 61,17-61,27 ppm (C15-22) ; 52,9 ppm (C27) ; 51,31-51,91 ppm (C29) ; 38,0 ppm (C23) ; 21,16-31,45 ppm (C2-12, C24-26) - 13,66 ppm (Cl). SM (ESI K⁺/MeOH), m/z : 643 (MK⁺, 100%) ; 473 (20%) ; 303 (7,5 %).
IR (cm⁻¹) : 3062 (OH) ; 2921 (CH) ; 1744 (CO) ; 1654 (HNCO) ; 1539 (NH); 1108 (COC).
Analyse Elémentaire :

| | % exp. | % calc. |
|---|---|---|
| C | 61,84 | (61,56) |
| H | 10,35 | (10,00) |
| N | 4,30 | (4,63) |
| O | 23,21 | (23,81) |

Dosage des traces d'ions par électrophorèse capillaire :

L'analyse des cations par électrophorèse capillaire en mode d'injection électrocinétique (étalon interne : ions NH₄⁺) montre que le produit contient une petite quantité d'ions Na⁺ : en moyenne 0,8% en moles (soit 0,03% en masse). Un autre lot de 4 g du composé **1a** a été synthétisé à partir du composé **8** en suivant le même mode opératoire pour les étapes 4 et 5. Ce lot contient 1,3% en moles d'ions Na⁺ (soit 0,05% en masse). Toutes les expériences de physico-chimie ont été menées sur ce dernier lot.

### Propriétés physico-chimiques du composé 1a :

### 1) Mesure de la densité

La densité du composé **1a,** déterminée à l'aide d'un densimètre Anton Paar, est de 0,950 à 25°C.

### 2) Détermination de la Concentration Micellaire Critique (cmc) et de la surface par tête polaire : Courbe γ=f(log C) obtenue par la méthode de l'anneau de Du Nouy.

La cmc et la surface par tête polaire du composé **1a** ont été déduites de mesures de tension superficielle réalisées sur un "Digital-Tensiometer K 10" de Krüss. Les résultats sont donnés dans le tableau 1 ci-dessous et comparés aux valeurs caractéristiques des tensioactifs non-ioniques C₁₂E₅ : C₁₂H₂₅-(OCH₂CH₂)₅-OH et C₁₂E₄ : C₁₂H₂₅-(OCH₂CH₂)₄-OH, tirées de la littérature ([10].

**Tableau 1 : Comparaison des cmc, γ_{cmc} et σ_{TA} de C₁₂E₄, C₁₂E₅ et du composé 1a**

| Composé | Cmc (mol.l⁻¹) | γ_{cmc} (mN.m⁻¹) | σ_{TA} ^{(c)} • (*A*²) |
|---|---|---|---|
| C₁₂E₅^{(a)} | 9,4 ± 0,5.10⁻⁵ | 29,2 | 51 ± 2 |
| C₁₂E₄(^{b}) | 10,6.10⁻⁵[10] | / | 45 [10] |
| **1a** | 4,9 ± 0,5.10⁻⁵ | 33,1 | 60 ± 2 |

| | | | |
|---|---|---|---|
| (a) valeurs déterminées expérimentalement ; (b) valeurs données dans la littérature [10] ; (c) : aire de la molécule à l'interface èau/air en (Angströms)². | | | |

### 3) Détermination de la température critiques T_{c} du composé 1a : Courbes de démixtion des systèmes eau/tensioactif

Les courbes de démixtion des systèmes eau/**1a** et eau/C₁₅E₅ ont été établies. Le composé **1a** présente une température critique de 57 ± 1°C.

Pour comparaison, les tensioactifs non-ioniques C₁₂E₄ et C₁₂E₅ ont une température critique de 6°C [5] et 29°C. La fonctionnalisation augmente donc considérablement 1a température critique par rapport à C₁₂E₄ mettant ainsi en évidence une forte contribution hydrophile du résidu amino-acide greffé.

### 4) Influence de la force ionique sur la courbe de démixtion du système eau/composé la

L'influence de la force ionique sur la température critique de **1a** a été déterminée en établissant les courbes de démixtion de solutions aqueuses de **1a** en présence de concentrations variables en LiNO₃.

La figure 1 qui illustre la variation de la température critique T_{c} (en °C) en fonction de la concentration massique en **1a** (%), montre que la température critique augmente avec la concentration en LiNO₃ et varie peu avec la concentration de **1a.**

Le tableau 2 permet de comparer les températures critiques T_{c} à différentes concentrations en LiNO₃ pour une concentration fixée en **1a** (4,9.10⁻² mol.L⁻¹ soit environ 3% en masse).

**Tableau 2**

| [LiNO₃](M) | Tc (°C) |
|---|---|
| 4,4 | 74,9 |
| 2 | 71,5 |
| 1 | 69,3 |
| 0,63 | 68,6 |
| 0,37 | 62,9 |
| 0 | 60,6 |

L'augmentation de T_{c}, "effet de salting-in" en présence de LiNO₃, est un effet déjà connu, caractéristique des tensioactifs de type CᵢEⱼ [11]. Aux faibles concentrations en LiNO₃, l'effet est peu marqué. Aux plus fortes concentrations, à partir de 0,6 M, cet effet devient plus important et on a alors une augmentation de la température critique T_{c} à peu près proportionnelle à la concentration en LiNO₃.

### Exemple 2 : Synthèse du composé 2a

Le composé **2a** est obtenu en 2 étapes à partir du tensioactif C₁₂E₅ (composé **11**) selon le schéma suivant :

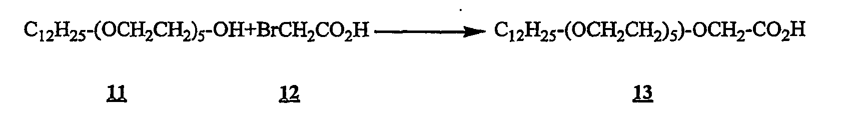

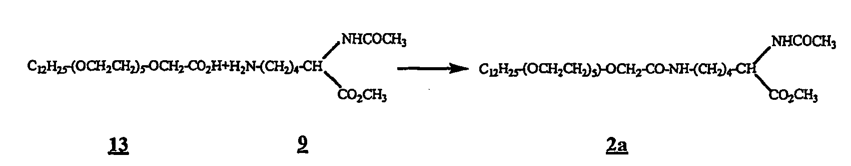

Dans la première étape, le tensioactif **11** C₁₂E₅ est transformé en acide **13** par réaction avec l'acide bromoacétique **12.** Le composé **2b** est ensuite obtenu par couplage de l'acide **13** sur l'ester méthylique de la N-acétyllysine **9.** Le rendement global de la synthèse par rapport au C₁₂E₅ est d'environ 65%.

### • Préparation du composé 9 : Ac-Lys(NH₂)-OMe (ce composé est accessible commercialement)

Dans un ballon bicol de 100 ml contenant 20 ml de méthanol sec refroidi à 0°C, 1,67 g de SOCl₂ (1,4.10⁻² mol) sont additionnés sous courant d'azote. 0,74 g de lysine N-acétylée (Ac-Lys(NH₂)-OH, 4.10⁻³ mol) est rapidement introduit sous courant d'azote dans le milieu réactionnel puis est laissé sous agitation pendant 12 heures à température ambiante. L'avancement de la réaction est suivi par CCM avec comme éluant un mélange (Chloroforme/Méthanol/Acide acétique, 5/3/1) et comme agent de révélation la ninhydrine. Dans ces conditions la N-acéhyl lysine possède un Rf de 0,07 et l'ester **9** a un Rf de 0,43.

Avant d'isoler le produit, le milieu réactionnel est neutralisé par du NaHCO₃, puis l'excès de méthanol est évaporé au rotavapor. Le produit brut, obtenu alors, est ensuite dilué dans l'éthanol sec dans le but d'éliminer les sels résiduels par centrifugation. Après concentration du surnageant et séchage sous pression réduite, 0,647 g de produit **9** (3,2.10⁻³ mol) sont isolés.
Rendement : 80% Caractéristiques du composé **9 :**
Aspect huileux
MM = 202,2 g/mol 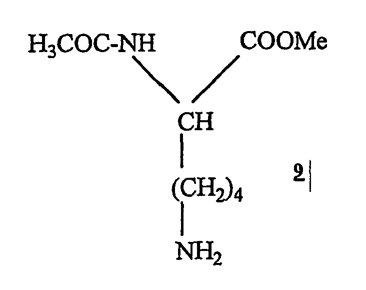 RMN ¹H (D₂O) _{:} 4,24-4,19 ppm (m, 1H, CH) ; 3,59 ppm (s, 3H, OCH₃) ; 2,825 ppm (t, 2H, -CH₂-CH₂-NH₂) ; 1, 87 ppm (s, 3H, -NHCOCH₃) ; 1,75-1,24 ppm (m, 6H, CH-(CH₂)₃-CH₂-NH₂).

### • étape : Préparation du composé 13 [C₁₂E₅-O-CH₂-COOH]

Dans un ballon tricol de 100 ml surmonté d'un réfrigérant et doté d'une arrivée d'azote, une suspension de NaH préalablement nettoyé de son huile minérale (3,45.10⁻² mol, 60% massique : 1,38 g dilué dans 10 ml de THF anhydre) est introduite sous courant d'azote. Le composé **11** C₁₂E₅ (9,86.10⁻³ mol, 4 g), dilué dans 10 ml de THF anhydre est additionné sous courant d'azote. Le milieu réactionnel est laissé à température ambiante sous agitation pendant 30 minutes sous courant d'azote. L'acide bromoacétique **12** (1,48.10⁻² mol, 2,07g) dilué dans 7 ml de THF anhydre est ensuite introduit progressivement à l'aide d'une ampoule à brome. Le temps d'addition s'étend sur 30 minutes pour pallier au dégagement thermique induit par la réaction de substitution. Le milieu réactionnel est ensuite chauffé à 60°C pendant 12 heures. L'avancement de la réaction est suivi par CCM avec comme éluant un mélange AcOEt/MeOH (4/1) et comme agent de révélation H₂SO₄ : Rf de **11** (C₁₂E₅) = 0,8 et Rf de **13** = 0,2). La réaction terminée, le THF est évaporé au rotavapor et le mélange brut repris à l'éther. Une extraction éther/acide chlorhydrique 1N permet, après 3 lavages de la phase organique, séchage de cette dernière sur MgSO₄ et évaporation de l'éther, de récupérer 4,30 g (9,26.10⁻³ mol) de produit **13,** débarrassé de toute impureté.
Rendement : 94%
Caractéristiques du produit **13 :**

C₁₂H₂₅-O-(CH₂CH₂O)₅-CH₂COOH **13**

Huile jaune
MM : 464 g/mol
RMN¹H CDCl₃ : 4,09 ppm (s, 2H, C₁₂E₅-O-CH₂-COOH) ; 3,66-3,52 ppm (m, 20H, O-(CH₂-CH₂)₅-O) ; 3,39 ppm (t, 2H, C₁₁H₂₃-CH₂-O-) ; 1,5 ppm (m, 2H, C₁₀H₂₁-CH₂-CH₂-O) ; 1,19 ppm (s, 18H, C₉H₁₈) ; 0,81 ppm (t, 3H, CH₃).

### • 2ème étape : Préparation du composé 2a

Dans un ballon bicol de 250 ml, muni d'un barreau aimanté et d'une arrivée d'azote, 1,22 g du composé **13** (2,637.10⁻³ mol) sont dissous dans 100 ml de DMF sec. 1,246 g de 1-hydroxybenzotriazole HOBT (9,232.10⁻³ mol) puis 0,64 g du composé **9** (3,16.10⁻³ mol) sont ajoutés sous courant d'azote. Après 15 minutes d'agitation à température ambiante, 1,265 g d'hydrochlorhydrate de N-(3-dimethylaminopropyl)-N'-ethylcarbodiimide EDC (6,6.10⁻³ mol) sont introduits. Le milieu réactionnel est ensuite laissé sous agitation pendant 12 heures à température ambiante. Le suivi de la réaction se fait par CCM avec un éluant AcOEt/MeOH 1/1 et H₂SO₄ comme agent de révélation. Dans ces conditions l'acide **13** possède un Rf de 0,3 et le produit **2a** a un Rf de 0,7. Après évaporation du DMF, le brut est repris par du dichlorométhane et lavé 3 fois avec HCl 1N. Après séchage sur MgSO₄ et évaporation du dichlorométhane, on obtient 1,56 g de produit **2a** brut (Rdt : 91%). Le produit **2a** est purifié par chromatographie sur colonne de silice (diamètre 3,5 cm, 80 g de SiO₂) avec un éluant AcOEt/MeOH 6/1. 1,2 g (1.851.10⁻³ mol) de produit **2a** pur sont récupérés.
Rendement : 70%
Caractéristiques du produit **2a :**
Huile possédant des reflets jaunes.
MM : 648,2 g/mol 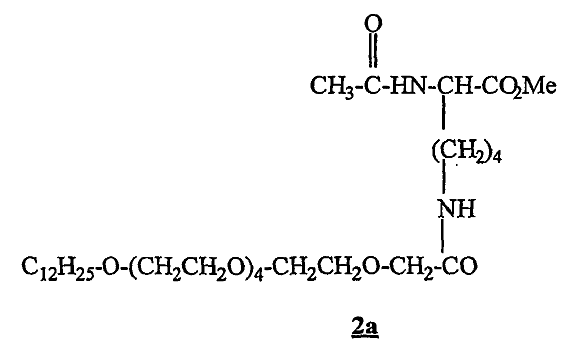 RMN¹H CDCl₃ : 7,10 ppm (t, 1H, CH₂CO-NH-(CH₂)₄-) ; 6,33 ppm (d, 1H, CH₃CO-NH-CH-) ; 4,56-4,50 ppm (m, 1H, (-HN-CH(CH₂)₄-CO₂Me) ; 3,98 ppm (s, 2H, C₁₂E₅-O-CH₂-CO(NH)-) ; 3,73 ppm (s, 3H, OMe) ; 3,66-3,55 ppm (m, 20H, O-(CH₂-CH₂)₅-O) ; 3,44 ppm (t, 2H, C₁₁H₂₃-CH₂-O-) ; 3,28 ppm (m, 2H, -CO-NH-CH₂-(CH₂)₃-) ; 2,02 ppm (s, 3H, CH₃-CO-NH-) : 1,80-1,25 ppm (m, 26H, -CH-(CH₂)₃-CH₂-NH₂, C₁₀H₂₁-CH₂-CH₂-O-, H₃C-C₉H₁₈-CH₂-) ; 0,87 ppm (t, 3H, H₃C-C₉H₁₈-CH₂-) .

### Exemple 3 : Synthèse du composé 2b :

Le composé **2b** est obtenu en 3 étapes à partir du tensioactif C₁₂E₅, composé **11,** selon le schéma suivant :

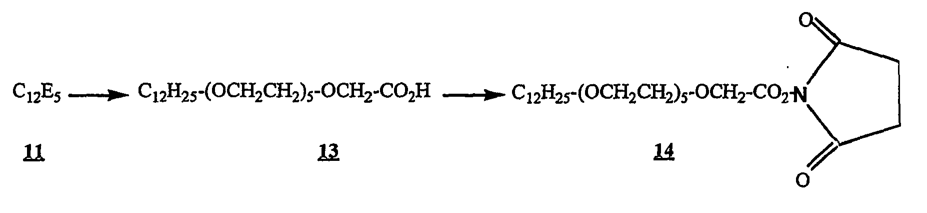

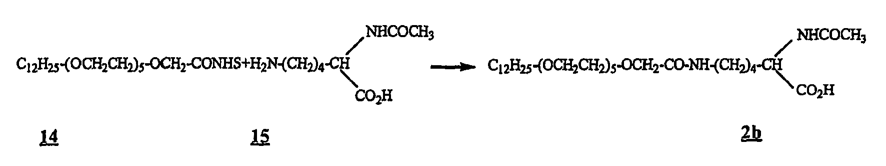

La première étape consiste en la transformation du tensioactif C₁₂E₅ **11** en acide **13** selon le protocole décrit précédemment (synthèse de 2a). L'acide **13** est activé sous forme d'ester de la N-hydroxysuccinimide **14.** Enfin la troisième étape, qui conduit à **2b,** consiste en un couplage de l'ester activé **14** sur la N-acétylLysine **15.** Le rendement global de la synthèse par rapport au C₁₂E₅ est de 80%.

### • 1ère étape : Préparation du composé 14 [C₁₂E₅-O-CH₂-CO-NES]

Dans un ballon bicol de 100 ml doté d'une sortie d'azote et d'une agitation magnétique, 4,18 g du composé **13** (9.10⁻³ mol) sont dissous dans 40 ml de CH₂Cl₂ anhydre. La température du milieu réactionnel étant abaissée à 0°C, 2 g d'EDC (1,08.10⁻² mol) sont introduits sous courant d'azote. Après 30 minutes d'agitation à 0°C, la N-hydroxysuccinimide (1,35.10⁻² mol, 1 554 g) est introduite. Le milieu réactionnel est ensuite agité 1 heure à 0°C puis 12 heures à température ambiante. Le suivi de la réaction est réalisé par CCM avec un éluant AcOEt/MeOH 2/1 et comme agent de révélation H₂SO₄ : les Rf sont respectivement de 0,12 pour le composé **11** et de 0,8 pour le composé **14.** Le milieu réactionnel est lavé 2 fois avec HCl 1N. La phase organique est séchée sur MgSO₄ puis concentrée au rotavapor. L'ester activé **14** est obtenu avec un rendement de 91% (8,2.10⁻³ mol, 4,59 g) et utilisé sans purification supplémentaire dans l'étape suivante. Caractéristiques du produit **14 :**
Huile jaune

MM : 561 g/mol 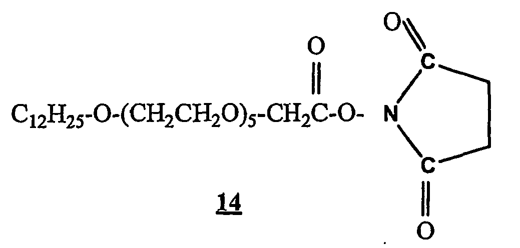 RMN ¹H (CDCl₃) : 4,47 ppm (s, 2H, O-CH₂-COON-) ; 3,75-3,52 ppm (m, 20H, O-(CH₂-CH₂)₅-O) ; 3,40 ppm (t, 2H, C₁₁H₂₃-CH₂-O-) ; 2,80 ppm (s, 4H, N-CO-CH₂-CH₂-CO) ; 1,5 ppm (m, 2H, C₁₀H₂₁CH₂-CH₂-O-) ; 1,21 ppm (s, 18H, H₃C-C₉H₁₈) ; 0,82 ppm (t, 3H, CH₃).

On observe sur le spectre la présence d'une impureté à 2,82 ppm en petite quantité.
IR : (cm⁻¹) : 1741 (C=O ester) ; 1784,10 (C=0 imide)

### • 2ème étape : Préparation du composé 2b

L'ester **14** (4,06.10⁻³ mol, 2,28 g) est dissous dans 60 ml d'acétone. La N-acétyl-Lysine **15** (6,1.10⁻³ mol, 1,148 g) et la triéthylamine (2,03.10⁻² mol, 2,054 g) en solution dans 25 ml d'eau ultra pure, sont ajoutés au milieu réactionnel à température ambiante. Après 12 heures de réaction à température ambiante, l'acétone est évaporée, puis le mélange est repris avec du dichlorométhane. Une extraction HCl 1N/CH₂Cl₂ permet d'éliminer tous les produits résiduels (excès de Ac-Lys-(OH), triéthylamine, N-hydroxysuccinimide). La phase organique est alors séchée sur MgSO₄ et le solvant éliminé sous pression réduite.

2,445 g de composé thermosensible **2b** (3,857.10⁻³ mol) sont obtenus.
Rendement = 95%.
Caractéristiques du composé **2b :**
Huile jaune
MM : 634,2 g/mol

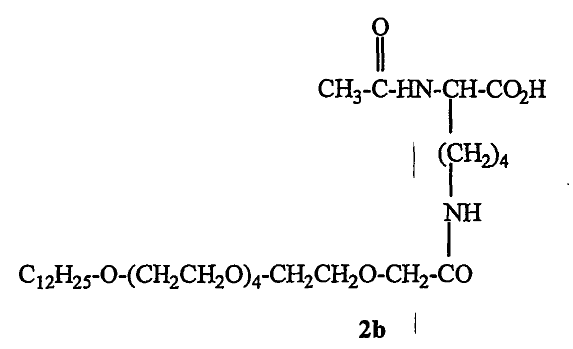

RMN ¹H (CDCl₃) _{:} 7,39 ppm (t, 1H, CH₂-CO-NH-(CH₂)₄-) ; 6,81 ppm (d, 1H, CH₃CO-NH-CH-) ; 4,58 ppm (m, 1H, (-HN-CH(CH₂)₄-CO₂H) ; 4,02 ppm (s, 2H, C₁₂E₅-O-CH₂-CO(NH)-) ; 3,76-3,57 ppm (20H, O-(CH₂-CH₂)₅-O) ; 3,45 ppm (t, 2H, C₁₀H₂₀-CH₂-O-) ; 3,36 ppm (m, 2H, -CO-NH-CH₂-(CH₂)₃-) ; 2,05 ppm (s, 3H, CH₃-CO-) ; 1,87-1,22 ppm (m, 26H, -CH-(CH₂)₃-CH₂-NH₂, C₁₀H₂₁-CH₂-CH₂-O-, H₃C-C₉H₁₈-CH₂-) ; 0,88 ppm (t, 3H, CH₃-CH₂-). On observe sur le spectre la présence d'une impureté à 2,85 ppm en petite quantité.

### Températures critiques des composés 2a et 2b :

Les températures critiques des composés **2a** et **2b** sont respectivement de 38 et 40°C : elles sont légèrement supérieures à celle du tensioactif non ionique greffé C₁₂E₅ (30°C).

Les valeurs des températures critiques des composés **2a** et **2b** dans l'eau et en présence de nitrates alcalins (NaNO₃ ou LiNO₃) sont répertoriées dans le tableau 3 ci-dessous :

**Tableau 3**

| Composé | Concentration massique | Concentration M | [NaNO₃] M | [LiNO₃] M | Tc (°C) ±/-2°C |
|---|---|---|---|---|---|
| 2a | 2,6% | 0,04 | 0 | 0 | 38 |
| 2a | 2,3% | 0,036 | 0 | 4 | 66 |
| 2a | 3% | 0,046 | 1 | 0 | 48 |
| 2a | 3% | 0,046 | 2 | 0 | 35 |
| 2h | 3% | 0,047 | 0 | 0 | 40 |
| 2b | 3% | 0,047 | 1 | 0 | 39 |
| 2h | 3% | 0,047 | 2 | 0 | 35 |

### Exemple 4 : Extraction de l'uranium, sous forme de nitrate d'uranyle, U^{VI}, par les composés 1a et 2a par variation de température

Les expériences sont réalisées en introduisant en solution aqueuse le composé hydrosoluble, thermosensible et thermoréversible **(1a** ou **2a)** du nitrate de lithium et du nitrate d'uranyle en concentrations respectives variables. La température critique est déterminée pour chaque composition. L'élévation de la température légèrement au-dessus du la température critique T_{c} (quelques degrés) provoque la séparation des deux phases : une phase concentrée de faible volume enrichie en ion uranyle et de densité supérieure à l'eau qui sédimente et une phase diluée surnageante appauvrie en ion uranyle. Après décantation naturelle, la teneur en U^{(VI)} restant dans la phase surnageante est déterminée.

Les concentrations de nitrate d'uranyle sont déterminées en phase aqueuse avant et après la température critique par fluorescence X (raie située entre 11,8 et 14,8 keV).

Les résultats des expériences sont répertoriés dans le tableau 4. Ils montrent que les composés thermosensibles **1a** et **2a** permettent de séparer efficacement l'ion uranyle par simple variation de température avec des rendements d'extraction allant jusqu'à 45% et cela en présence de quantités stoechiométriques ou sub-stoechiométriques de composé **1a** ou **2a.** En une seule étape de séparation, les capacités d'extraction atteignent 0,35-0,4 mole d'U par mole de composé thermosensible.

A titre comparatif, on a donné également dans le tableau 4, les résultats obtenus avec le tensioactif non-ionique simple C₁₂E₅ et avec un mélange du tensioactif non-ionique C₁₂E₅ et d'un dérivé de lysine, l'ester méthylique de la lysine N,N'-diacétylée LysNAcOMe.

Dans ces deux cas, la séparation de l'uranium est négligeable. Ceci met clairement en évidence l'intérêt des composés thermosensibles de l'invention et de leur structure en deux parties ayant des fonctions différentes, la première partie permettant d'assurer aisément la séparation et la seconde partie assurant la complexation de l'espèce chimique à séparer.

Les résultats du tableau 4 montrent également que le composé **1a,** à chaîne polyéthoxylée pendante avec un groupement hydroxy libre, a des capacités d'extraction supérieures à celles du composé **2a,** pour lequel le résidu acide aminé complexant est greffé par l'extrémité du groupement polyéthoxylé.

Pour une concentration fixée en nitrate de lithium, les températures de séparation de phase (point de trouble) diminuent en présence de cation uranyle avec un effet d'autant plus important que le rapport uranyle/composé thermosensible augmente. De plus, la comparaison des températures critiques et des quantités d'uranium extrait pour les composés **1a** et **2a** montre que la diminution de la température critique est d'autant plus importante que la quantité d'uranium extrait est grande : l'abaissement de T_{c} semble donc résulter de la complexation du cation uranyle par le composé.

**Tableau 4**

| | **Composé concentration (M)** | **[LiNO₃] M** | **[UO₂²⁺]ᵢₙᵢₜ^{(a)}** | **T_{c} °C** | **[UO₂²⁺]ᵢₙᵢₜ^{(b)} M** | **U extrait^{(c)} %** | **U mol/mol** | **extrait/composé^{(d)} mol/g** |
|---|---|---|---|---|---|---|---|---|
| **I** | **1a :** 0,04 M | 0 | 0 | 70 | - | - | - | - |
| **II** | **1a :** 0,0044 M | 4 | 0 | 77 | - | - | - | - |
| **III** | **1a :** 0,004 M | 4 | 0,021 | 26 | 0,012 | 43% | 0,22 | 3,7.10⁻⁴ |
| **IV** | **1a :** 0,043 M | 4 | 0,028 | 24 | 0,018 | 36% | 0,23 | 3,9.10⁻⁴ |
| **V** | **1a** : 0,04 M | 4 | 0,041 | ∼0 | 0,026 | 36% | 0,37 | 6,2.10⁻⁴ |
| **XI** | **1a :** 0,04 M | 2 | 0,022 | <80 | 0,012 | 45% | 0,25 | 4,1.10⁻⁴ |
| | | | | | | | | |
| **VII** | **2a :** 0,04 M | 0 | 0 | 38 | - | - | - | - |
| **VIII** | **2a :** 0,036 M | 4 | 0 | 66 | - | - | - | - |
| **IX** | **2a :** 0,043 M | 4 | 0,021 | 51 | 0,016 | 24% | 0,12 | 1,8.10⁻⁴ |
| **X** | **2a :** 0,042 M | 4 | 0,038 | 41 | 0,032 | 16% | 0,14 | 2,2.10⁻⁴ |
| **Test de référence** | | | | | | | | |
| **XI** | **C₁₂E₅^{(e)} :** 0,04 M | 2 | 0,027 | 38 | 0,026 | <3% | <0,025 | <6.10⁻³ |
| **XII** | **C₁₂O₅ :** 0,04 M+ **LysNAcOMe^{(f)} :** 0,04 M | 2 | 0,22 | 38 | 0,022 | ∼ 0 | ∼ 0 | ∼ 0 |

| | | | | | | | | |
|---|---|---|---|---|---|---|---|---|
| (a) : concentration initiale en nitrate d'uranyle ; (b) : concentration en uranyle restant dans la phase surnageante après séparation ; (c) : pourcentage en uranyle extrait calculé en négligeant les variations de volume : [U]init-[U]restant/[U]init ; (d) : exprimé par rapport à la quantité de composé introduit initialement ; (e) : C₁₂E₅ : C₁₂H₂₅-(OCH₂CH₂)₅-OH (f) : LysNAcOMe : ester méthylique de la lysine N,N'-diacétylée | | | | | | | | |

### REFERENCES CITEES

- [1]: Bergbreiter D.E., Koshti N., Franchina J.G., Frels J.G., Angew. Chem. Int. Ed., 39, 2000, 1040-2,
- [2]: Takeshita K. et Nakano Y., Solvent Extraction and Ion Exchange, 18(2), 2000, 375-86,
- [3]: Hinze W.L., Pramauro E., Critical Reviews in Analytical Chemistry, 24(2), 1993, 133-77,
- [4]: Tani H., Kamidate T., Watanabe H., J. Chromatogr. A, 780, 1997, 229-241,
- [5]: Huibers P.D.T., Shah D.O., Katrizky A.R., J. Colloid and Interface Sci., 193, 1997, 132-6,
- [6]: Philippon A., Tao J., Tétard D., Degueil-Castaing M, Maillard B., Synthetic Commun., 27(15), 1997,2651-2682.
- [7]: Wassermann H., Azim E., Coudert G., Achilefii S., Selve C., J. Chem. Soc. Perkin Trans.2, 1992, 2043-2047.
- [8]: Popovitz Biro R., HilI K., Shavit E., Hung D.J., Lahav M., Leiserowitz L., Sagiv J., Hsiung H., Meredith G.R., Vanherzeele FL, J. Amer. Chem. Soc., 112, 1990, 2498-2506.
- [9]: P.J. Kocienski, Protecting Groups, Georg Thieme Verlag, Stuttgart, New York, 1994, pp.83-87.
- [10]: Grillo Isabelle., Thèse de l'Université Paris Xl (1998), p. 175.
- [11]: Schott H, Kyu Han S, J. Pharmaceutical Sci., 64 (4), 1975, 658-664.
- [12]: US-A-4,154,674.
- [13]: WO-A-00/73521.

## Revendications

1. Composé thermosensible ayant la propriété d'être soluble dans l'eau en dessous d'une température critique T_{c} et insoluble dans l'eau au-dessus de cette température T_{c}, cette propriété étant thermoréversible, **caractérisé en ce qu'**il comprend une première partie amphiphile et thermoréversible répondant à l'une des formules (I) et (II) suivantes : 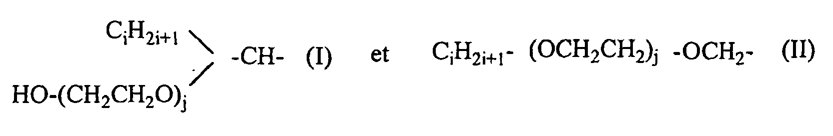 dans lesquelles i est un nombre entier allant de 1 à 20 et j est un nombre entier allant de 3 à 30, cette première partie étant liée chimiquement à une seconde partie capable de complexer un ion métallique.

2. Composé selon la revendication 1, dans lequel la seconde partie comprend un acide aminé, un peptide, un oligopeptide, un polypeptide, un oligomère ou un polymère comportant un ou plusieurs groupements complexants de type amine, amide ou acide.

3. Composé selon la revendication 2, dans lequel la seconde partie comprend un seul acide aminé.

4. Composé selon la revendication 3, dans lequel l'acide aminé est la lysine.

5. Composé selon la revendication 4, dans lequel la seconde partie répond à la formule (III) suivante : 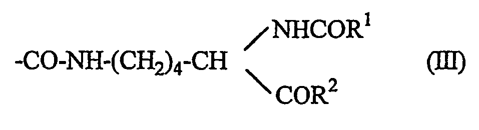 dans laquelle R¹ représente un groupe hydrocarboné et R² représente un groupe hydroxyle, un groupe alkoxy ou un groupement azoté, substitué ou non.

6. Composé selon la revendication 5, dans lequel R¹ représente le groupe méthyle et R² représente le groupe hydroxyle ou le groupe méthoxy.

7. Composé répondant à la formule (IV) suivante : 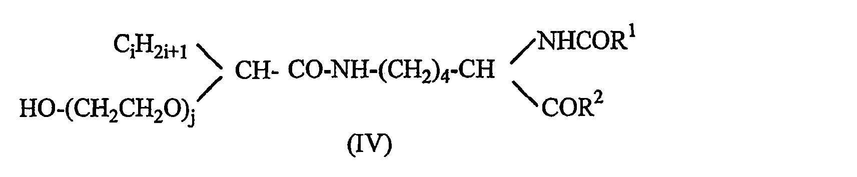 dans laquelle i est égal à 12, j est égal à 4, R¹ représente CH₃ et R² représente OCH₃.

8. Composé répondant à la formule (V) suivante : 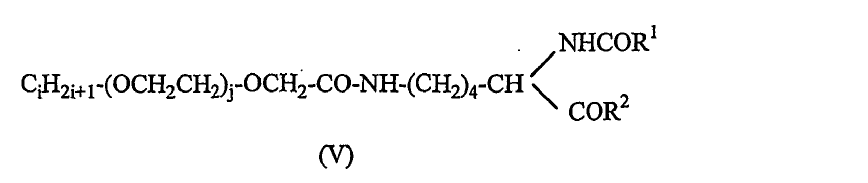 dans laquelle i est égal à 12, j est égal à 5, R¹ représente CH₃ et R² représente OCH₃ ou OH.

9. Composé selon l'une quelconque des revendications 1 à 8, présentant une température critique T_{c} de 20 à 90°C, à une concentration massique de 0,1 à 25% en solution aqueuse.

10. Composé selon la revendication 7, présentant une température critique T_{c} de 55 à 75°C, à une concentration massique de 0,5 à 25% en solution aqueuse.

11. Composé selon la revendication 8, présentant une température critique T_{c} de 30 à 70°C, à une concentration massique de 0,1 à 25% en solution aqueuse.

12. Procédé de préparation d'un composé thermosensible, ayant la propriété d'être soluble dans l'eau en dessous d'une température critique T_{c} et insoluble dans l'eau au dessus de cette température T_{c}, cette propriété étant thermoréversible, **caractérisé en ce qu'**il comprend la réaction d'un dérivé carboxylique répondant à l'une des formules (VI) et (VIII) suivantes : 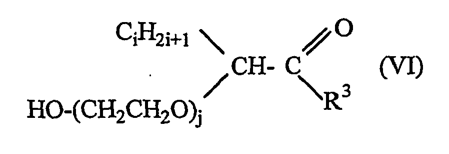 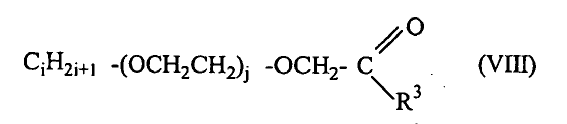 dans lesquelles i est un nombre entier allant de 1 à 20, j est un nombre entier allant de 3 à 30 et R³ représente Cl, OH ou un groupe alkoxy, avec une amine ou une polyamine primaire ou secondaire, comportant un groupe capable de complexer un ion métallique.

13. Procédé de préparation d'un composé de formule (IV) suivante : 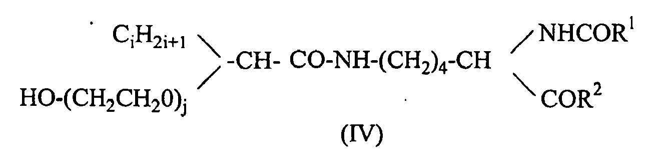 dans laquelle i est un nombre entier allant de 1 à 20, j est un nombre entier allant de 3 à 30, R¹ représente un groupe hydrocarboné et R² représente un groupe hydroxyle, un groupe alkoxy ou un groupement azoté, substitué ou non,
qui comprend la réaction d'un dérivé carboxylique de formule suivante (VI) : 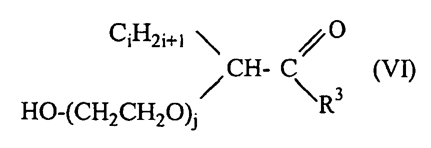 dans laquelle i et j sont tels que définis ci-dessus, et R³ représente Cl, OH ou un groupe alkoxy, avec le dérivé de lysine de formule(VII) suivante : 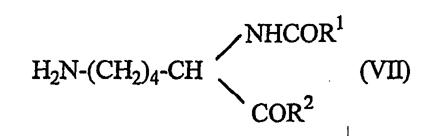 dans laquelle R¹ et R² sont tels que définis ci-dessus.

14. Procédé selon la revendication 13, dans lequel le groupe hydroxyle du dérivé carboxylique de formule (VI) est protégé et le groupe carboxylique de ce dérivé est activé avant de le faire réagir avec le dérivé de lysine de formule (VII).

15. Procédé selon la revendication 14, dans lequel le groupe hydroxyle est protégé par le tétrahydropyranne, et le groupe carboxylique est activé par la N-hydroxysuccinimide.

16. Procédé selon la revendication 14 ou 15, qui comprend en outre une étape de déprotection du groupe hydroxyle du dérivé de formule (VI) après réaction de celui-ci avec le dérivé de lysine de formule (VII).

17. Procédé de préparation d'un composé répondant à la formule (V) suivante : 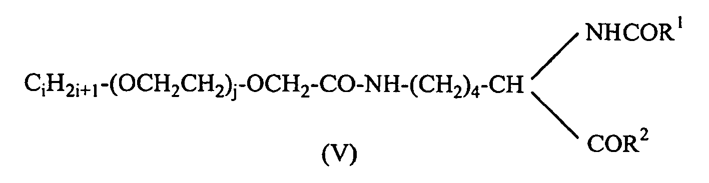 dans laquelle i est un nombre entier allant de 1 à 20, j est un nombre entier allant de 3 à 30, R¹ représente un groupe hydrocarboné et R² représente un groupe hydroxyle, un groupe alkoxy ou un groupement azoté, substitué ou non,
qui comprend la réaction d'un dérivé carboxylique de formule (VIII) suivante : 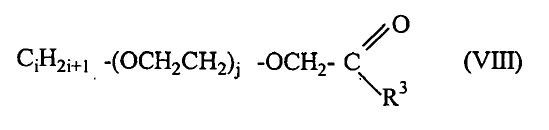 dans laquelle i et j sont tels que définis ci-dessus, et R³ représente Cl, OH ou un groupe alkoxy, avec le dérivé de lysine de formule(VII) suivante : 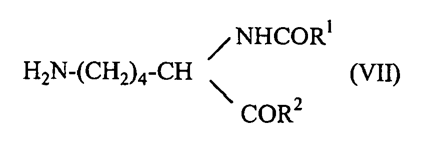 dans laquelle R¹ et R² sont tels que définis ci-dessus.

18. Procédé d'extraction d'au moins un ion métallique présente dans une solution aqueuse, qui comprend les étapes suivantes :
a) ajouter à la solution aqueuse un composé thermosensible selon l'une quelconque des revendications 1 à 11, à une température inférieure à la température critique T_{c} de ce composé,
b) porter le milieu réactionnel à une température supérieure à la température critique T_{c} du composé pour séparer le milieu réactionnel en deux phases constituées respectivement par une phase concentrée enrichie en l'ion métallique à extraire, de densité supérieure à celle de l'eau, qui sédimente, et en une phase aqueuse diluée surnageante appauvrie en l'ion métallique à extraire, et
c) séparer la phase concentrée en l'ion métallique du milieu réactionnel.

19. Procédé d'extraction d'au moins un ion métallique présente dans une solution aqueuse, qui comprend les étapes suivantes :
a) ajouter à la solution aqueuse un composé thermosensible selon l'une quelconque des revendications 1 à 11, et une phase organique immiscible à l'eau, à une température inférieure à la température critique T_{c} de ce composé,
b) porter le milieu réactionnel à une température supérieure à la température critique T_{c} pour transférer le composé thermosensible et l'ion métallique à extraire dans la phase organique, et
c) séparer la phase organique contenant l'ion métallique du milieu réactionnel.

20. Procédé selon la revendication 18 ou 19, dans lequel la quantité de composé thermosensible ajoutée dans l'étape a) est telle que la concentration massique de la solution aqueuse en composé thermosensible soit de 0,1 à 25%.

21. Procédé selon l'une quelconque des revendications 18 à 20, dans lequel l'ion métallique à extraire est l'uranium qui est présent sous forme d'ion uranyle dans la solution aqueuse.

22. Procédé selon la revendication 21, dans lequel le composé thermosensible répond à la formule (IV) suivante : 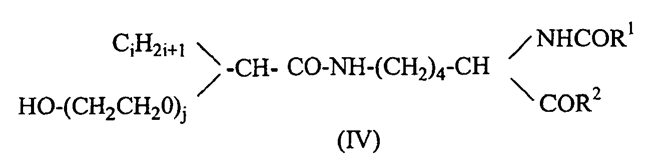 dans laquelle i est égal à 12, j est égal à 4, R¹ représente CH₃ et R² représente OCH₃.

## Claims

1. Heat-sensitive compound having the property of being soluble in water below a critical temperature T_{c} and insoluble in water above this temperature T_{c}, this property being thermally reversible, **characterized in that** it comprises a first amphiphilic and thermally reversible part corresponding to one of the following formulae (I) and (II): 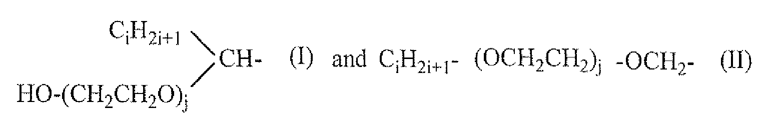 in which i is an integer ranging from 1 to 20 and j is an integer ranging from 3 to 30, this first part being chemically bonded to a second part capable of complexing a metal ion.

2. Compound according to Claim 1, in which the second part comprises an amino acid, a peptide, an oligopeptide, a polypeptide, an oligomer or a polymer comprising one or more complexing groups of amine, amide or acid type.

3. Compound according to Claim 2, in which the second part comprises a single amino acid.

4. Compound according to Claim 3, in which the amino acid is lysine.

5. Compound according to Claim 4, in which the second part corresponds to the following formula (III): 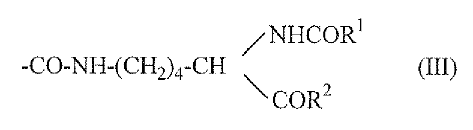 in which R¹ represents a hydrocarbonaceous group and R² represents a hydroxyl group, an alkoxy group or a nitrogenous group which may or may not be substituted.

6. Compound according to Claim 5, in which R¹ represents the methyl group and R² represents the hydroxyl group or the methoxy group.

7. Compound corresponding to the following formula (IV): 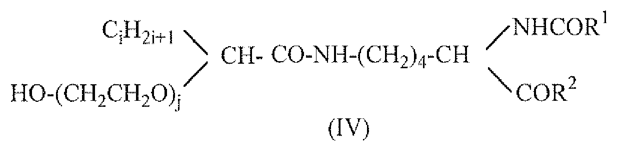 in which i is equal to 12, j is equal to 4, R¹ represents CH₃ and R² represents OCH₃.

8. Compound corresponding to the following formula (V): 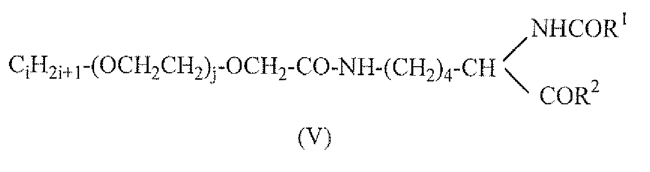 in which i is equal to 12, j is equal to 5, R¹ represents CH₃ and R² represents OCH₃ or OH.

9. Compound according to any one of Claims 1 to 8, exhibiting a critical temperature T_{c} of 20 to 90°C at a concentration by weight of 0.1 to 25% in aqueous solution.

10. Compound according to Claim 7, exhibiting a critical temperature T_{c} of 55 to 75°C at a concentration by weight of 0.5 to 25% in aqueous solution.

11. Compound according to Claim 8, exhibiting a critical temperature T_{c} of 30 to 70°C at a concentration by weight of 0.1 to 25% in aqueous solution.

12. Process for the preparation of a heat-sensitive compound having the property of being soluble in water below a critical temperature T_{c} and insoluble in water above this temperature T_{c}, this property being thermally reversible, **characterized in that** it comprises the reaction of a carboxylic derivative corresponding to one of the following formulae (VI) and (VIII): 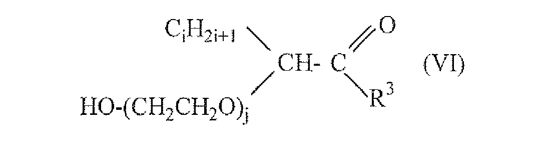 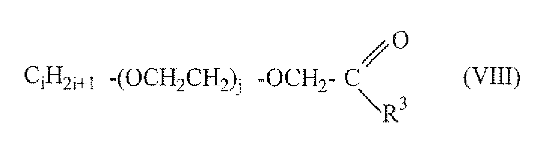 in which i is an integer ranging from 1 to 20, j is an integer ranging from 3 to 10 and R³ represents Cl, OH or an alkoxy group, with a primary or secondary amine or polyamine comprising a group capable of complexing a metal ion.

13. Process for the preparation of a compound of following formula (IV): 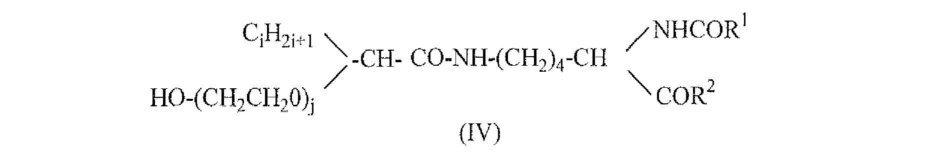 in which i is an integer ranging from 1 to 20, j is an integer ranging from 3 to 30, R¹ represents a hydrocarbonaceous group and R² represents a hydroxyl group, an alkoxy group or a nitrogenous group which may or may not be substituted,
which comprises the reaction of a carboxylic derivative of following formula (VI): 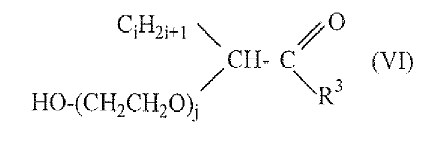 in which i and j are as defined above and R³ represents Cl, OH or an alkoxy group, with the lysine derivative of following formula (VII): 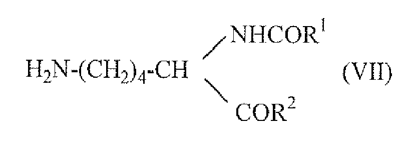 in which R¹ and R² are as defined above.

14. Process according to Claim 13, in which the hydroxyl group of the carboxylic derivative of formula (VI) is protected and the carboxyl group of this derivative is activated before reacting it with the lysine derivative of formula (VII).

15. Process according to Claim 14, in which the hydroxyl group is protected with tetrahydropyran and the carboxyl group is activated with N-hydroxysuccinimide.

16. Process according to Claim 14 or 15, which additionally comprises a stage of deprotection of the hydroxyl group of the derivative of formula (VI) after reaction of the latter with the lysine derivative of formula (VII).

17. Process for the preparation of a compound corresponding to the following formula (V): 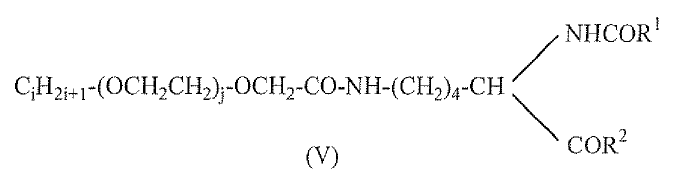 in which i is an integer ranging from 1 to 20, j is an integer ranging from 3 to 30, R¹ represents a hydrocarbonaceous group and R² represents a hydroxyl group, an alkoxy group or a nitrogenous group which may or may not be substituted,
which comprises the reaction of a carboxylic derivative of following formula (VIII): 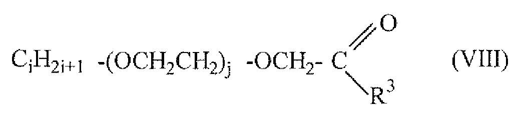 in which i and j are as defined above and R³ represents Cl, OH or an alkoxy group, with the lysine derivative of following formula (VII): 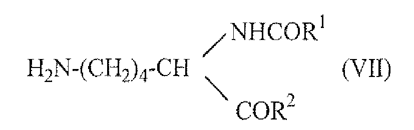 in which R¹ and R² are as defined above.

18. Process for the extraction of at least one metal ion present in an aqueous solution, which comprises the following stages:
a) a heat-sensitive compound according to any one of claims 1 to 11 is added to the aqueous solution at a temperature lower than the critical temperature T_{c} of this compound,
b) the reaction medium is brought to a temperature greater than the critical temperature T_{c} of the compound in order to separate the reaction medium into two phases, respectively composed of a concentrated phase enriched in the metal ion to be extracted, with a density greater than that of water, which sediments, and of a supernatant dilute aqueous phase depleted in the metal ion to be extracted, and
c) the phase concentrated in the metal ion is separated from the reaction medium.

19. Process for the extraction of at least one metal ion present in an aqueous solution, which comprises the following stages:
a) a heat-sensitive compound according to any one of Claims 1 to 11 and a water-immiscible organic phase are added to the aqueous solution at a temperature lower than the critical temperature T_{c} of this compound,
b) the reaction medium is brought to a temperature greater than the critical temperature T_{c} in order to transfer the heat-sensitive compound and the metal ion to be extracted into the organic phase, and
c) the organic phase comprising the metal ion is separated from the reaction medium.

20. Process according to Claim 18 or 19, in which the amount of heat-sensitive compound added in stage a) is such that the concentration by weight of heat-sensitive compound in the aqueous solution is from 0.1 to 25%.

21. Process according to any one of Claims 18 to 20, in which the metal ion to be extracted is uranium which is present in the form of the uranyl ion in the aqueous solution.

22. Process according to Claim 21, in which the heat-sensitive compound corresponds to the following formula (IV): 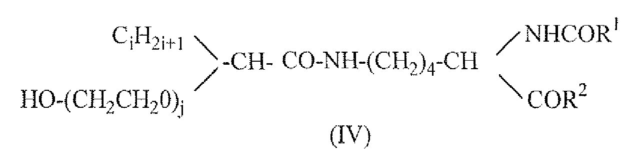 in which i is equal to 12, j is equal to 4, R¹ represents CH₃ and R² represents OCH₃.

## Patentansprüche

1. Wärmeempfindliche Verbindung mit der Eigenschaft, dass sie in Wasser unterhalb einer kritischen Temperatur T_{c} löslich ist und in Wasser oberhalb dieser Temperatur T_{c} unlöslich ist, wobei diese Eigenschaft thermisch reversibel ist, **dadurch gekennzeichnet, dass** sie einen ersten amphiphilen und thermisch reversiblen Teil umfasst, der einer der nachstehenden Formeln (I) und (II) entspricht: 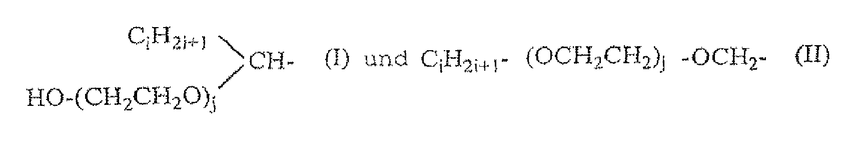 worin i eine ganze Zahl im Bereich von 1 bis 20 ist und j eine ganze Zahl im Bereich von 3 bis 30 ist, wobei der erste Teil chemisch an einen zweien Teil gebunden ist, der ein Metallion komplexieren kann.

2. Verbindung nach Anspruch 1, bei welcher der zweite Teil eine Aminosäure, ein Peptid, ein Oligopeptid, ein Polypeptid, ein Oligomer oder ein Polymer umfasst, die oder das eine oder mehrere komplexierende Gruppe(n) des Amin-, Amid- oder Säuretyps aufweist.

3. Verbindung nach Anspruch 2, bei welcher der zweite Teil eine einzelne Aminosäure umfasst.

4. Verbindung nach Anspruch 3, bei der die Aminosäure Lysin ist.

5. Verbindung nach Anspruch 4, bei welcher der zweite Teil der nachstehenden Formel (III) entspricht: 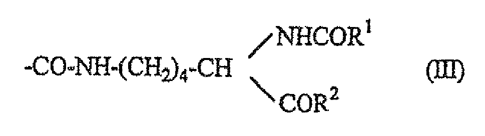 worin R¹ eine Kohlenwasserstoffgruppe darstellt und R² eine Hydroxylgruppe, eine Alkoxygruppe oder eine Stickstoff-enthaltende Gruppe darstellt, die substituiert oder unsubstituiert ist.

6. Verbindung nach Anspruch 5, bei der R¹ eine Methylgruppe darstellt und R² eine Hydroxylgruppe oder eine Methoxygruppe darstellt.

7. Verbindung, die der nachstehenden Formel (IV) entspricht: 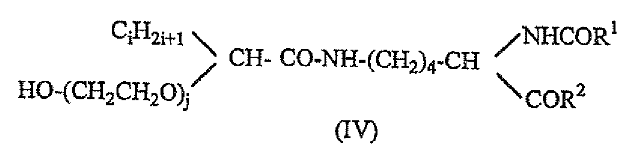 worin gleich 12 ist, j gleich 4 ist, R¹ CH₃ darstellt und R² OCH₃ darstellt.

8. Verbindung, die der nachstehenden Formel (V) entspricht: 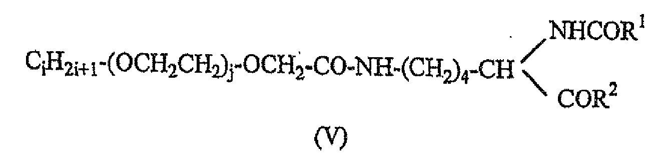 worin i gleich 12 ist, j gleich 5 ist, R¹ CH₃ darstellt und R² OCH₃ oder OH darstellt.

9. Verbindung nach einem der Ansprüche 1 bis 8, bei der die kritische Temperatur T_{c} 20 bis 90 °C beträgt, bei einer Konzentration von 0,1 bis 25 Massen-% in wässriger Lösung.

10. Verbindung nach Anspruch 7, bei der die kritische Temperatur T_{c} 55 bis 75 °C beträgt, bei einer Konzentration von 0,5 bis 25 Massen-% in wässriger Lösung.

11. Verbindung nach Anspruch 8, bei der die kritische Temperatur T_{c} 30 bis 70 °C beträgt, bei einer Konzentration von 0,1 bis 25 Massen-% in wässriger Lösung.

12. Verfahren zur Herstellung einer wärmeempfindlichen Verbindung mit der Eigenschaft, dass sie in Wasser unterhalb einer kritischen Temperatur T_{c} löslich ist und in Wasser oberhalb dieser Temperatur T_{c} unlöslich ist, wobei diese Eigenschaft thermisch reversibel ist, **dadurch gekennzeichnet, dass** das Verfahren die Umsetzung eines Carboxylderivats entsprechend einer der nachstehenden Formen (VI) und (VIII): 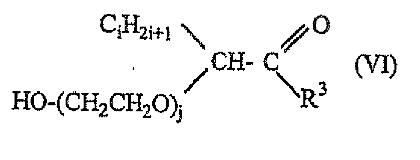 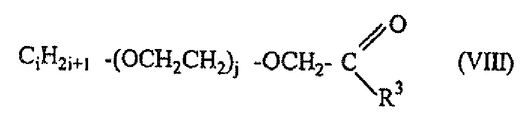 worin i eine ganze Zahl im Bereich von 1 bis 20 ist, j eine ganze Zahl im Bereich von 3 bis 30 ist und R³ Cl, OH oder eine Alkoxygruppe darstellt,
mit einem primären oder sekundären Amin oder Polyamin umfasst, das eine Gruppe aufweist, die ein Metallion komplexieren kann.

13. Verfahren zur Herstellung einer Verbindung der nachstehenden Formel (IV): 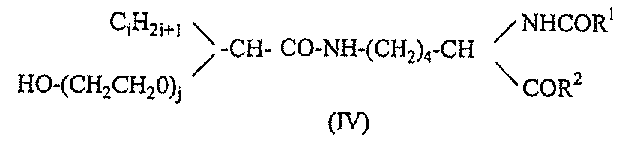 worin i eine ganze Zahl im Bereich von 1 bis 20 ist, j eine ganze Zahl im Bereich von 3 bis 30 ist, R¹ eine Kohlenwasserstoffgruppe darstellt und R² eine Hydroxylgruppe, eine Alkoxygruppe oder eine Stickstoff-enthaltende Gruppe darstellt, die substituiert oder unsubstituiert ist,
wobei das Verfahren die Umsetzung eines Carboxylderivats der nachstehenden Formel (VI): 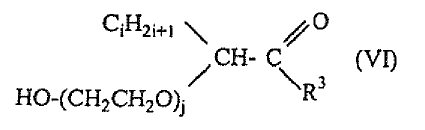 worin i und j die vorstehend angegebene Bedeutung haben und R³ Cl, OH oder eine Alkoxygruppe darstellt, mit einem Lysinderivat der nachstehenden Formel (VII) umfasst: 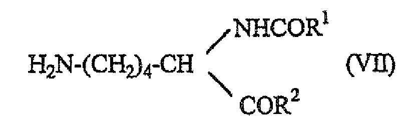 worin R¹ und R² die vorstehend angegebene Bedeutung haben.

14. Verfahren nach Anspruch 13, bei dem die Hydroxylgruppe des Carboxylderivats der Formel (VI) geschützt wird und die Carboxylgruppe dieses Derivats vor dem Umsetzen des Derivats mit dem Lysinderivat der Formel (VII) aktiviert wird.

15. Verfahren nach Anspruch 14, bei dem die Hydroxylgruppe mit Tetrahydropyran geschützt wird und die Carboxylgruppe mit N-Hydroxysuccinimid aktiviert wird.

16. Verfahren nach Anspruch 14 oder 15, das ferner den Schritt des Entschützens der Hydroxylgruppe des Derivats der Formel (VI) nach dem Umsetzen des Derivats der Formel (VI) mit dem Lysinderivat der Formel (VII) umfasst.

17. Verfahren zur Herstellung einer Verbindung der nachstehenden Formel (V): 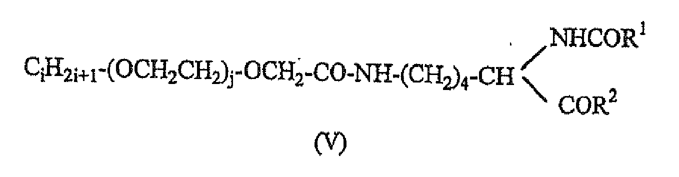 worin i eine ganze Zahl im Bereich von 1 bis 20 ist, j eine ganze Zahl im Bereich von 3 bis 30 ist, R¹ eine Kohlenwasserstoffgruppe darstellt und R² eine Hydroxylgruppe, eine Alkoxygruppe oder eine Stickstoff-enthaltende Gruppe darstellt, die substituiert oder unsubstituiert ist,
wobei das Verfahren die Umsetzung eines Carboxylderivats der nachstehenden Formel (VIII): 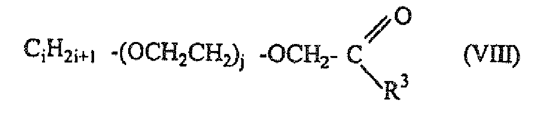 worin i und j die vorstehend angegebene Bedeutung haben und R³ Cl, OH oder eine Alkoxygruppe darstellt, mit einem Lysinderivat der nachstehenden Formel (VII) umfasst: 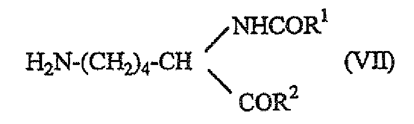 worin R¹ und R² die vorstehend angegebene Bedeutung haben.

18. Verfahren zur Extraktion mindestens eines Metallions, das in einer wässrigen Lösung vorliegt, welches die nachstehenden Schritte umfasst:
a) Zusetzen einer wärmeempfindlichen Verbindung nach einem der Ansprüche 1 bis 11 zu der wässrigen Lösung bei einer Temperatur unterhalb der kritischen Temperatur T_{c} der Verbindung,
b) Bringen des Reaktionsmediums auf eine Temperatur über der kritischen Temperatur T_{c} der Verbindung, um das Reaktionsmedium in zwei Phasen zu trennen, wobei die zwei Phasen aus einer konzentrierten Phase, die bezüglich des zu extrahierenden Metallions angereichert ist und die eine Dichte aufweist, die höher ist als die Dichte von Wasser, und die sedimentiert, und einer verdünnten, überstehenden wässrigen Phase, die bezüglich des zu extrahierenden Metallions abgereichert ist, zusammengesetzt sind, und
c) Abtrennen der Phase, die bezüglich des Metallion angereichert ist, von dem Reaktionsmedium.

19. Verfahren zur Extraktion mindestens eines Metallions, das in einer wässrigen Lösung vorliegt, welches die nachstehenden Schritte umfasst:
a) Zusetzen einer wärmeempfindlichen Verbindung nach einem der Ansprüche 1 bis 11 und einer mit Wasser nicht mischbaren organischen Phase zu der wässrigen Lösung bei einer Temperatur unterhalb der kritischen Temperatur T_{c} der Verbindung,
b) Bringen des Reaktionsmediums auf eine Temperatur über der kritischen Temperatur T_{c}, um die wärmeempfindliche Verbindung und das zu extrahierende Metallion in die organische Phase zu überführen, und
c) Abtrennen der organischen Phase, die das Metallion enthält, von dem Reaktionsmedium.

20. Verfahren nach Anspruch 18 oder 19, bei dem die Menge der im Schritt a) zugesetzten wärmeempfindlichen Verbindung derart ist, dass die Konzentration, bezogen auf die Masse, der wärmeempfindlichen Verbindung in der wässrigen Lösung 0,1 bis 25 % beträgt.

21. Verfahren nach einem der Ansprüche 18 bis 20, bei dem das zu extrahierende Metallion Uran ist, das in der Form von Uranylionen in der wässrigen Lösung vorliegt.

22. Verfahren nach Anspruch 21, bei dem die wärmeempfindliche Verbindung der nachstehenden Formel (IV) entspricht: 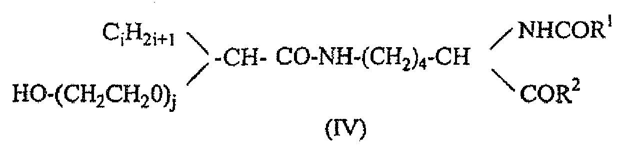 worin 1 gleich 12 ist, j gleich 4 ist, R¹ CH₃ darstellt und R² OCH₃ darstellt.
